# EUROPEAN PATENT SPECIFICATION

(11) **EP 3 890 826 B1**
(45) Date of publication and mention of the grant of the patent: **10.12.2025**
(21) Application number: 19827637.0
(22) Date of filing: 09.12.2019
(51) Int. Cl.: A61N 1/375, A61N 1/05, A61N 1/36

(54) **HERMETIC PACKAGING OF ELECTRONIC COMPONENTS**
HERMETISCHE VERPACKUNG VON ELEKTRONISCHEN KOMPONENTEN
EMBALLAGE HERMÉTIQUE POUR COMPOSANTS ÉLECTRONIQUES

(30) Priority: 07.12.2018 WO PCT/EP2018/084059
(43) Date of publication of application: 13.10.2021
(60) Divisional application: 25197741.9
(73) Proprietor: La Science SAS, 75012 Paris (FR)
(72) Inventor: DETERRE, Martin, 75012 Paris (FR); TENAILLEAU, Jean-René, 85710 La Garnache (FR); KAMINS, Theodore I, Palo Alto, California 94306 (US)
(74) Representative: Graf von Stosch Patentanwaltsgesellschaft mbH
(86) International application number: PCT/EP2019/084275
(87) International publication number: WO 2020/115332

(56) References cited:
- US-A1- 2011 039 050
- US-A1- 2013 330 498
- US-A1- 2016 120 472
- US-A1- 2016 284 853

## Description

The present invention relates to the field of electronic devices, in particular implantable electronic devices, e.g. for bio-medical applications, and more particularly, to hermetically packaged electronic devices for bio-medical *in vivo* applications and methods of packaging electronic components for manufacturing such electronic devices.

### BACKGROUND

Electronic devices are ubiquitously used and often required to function even under harsh environmental conditions. E.g., implantable electronic devices, such as chips, are used in bio-medical *in vivo* applications, e.g. for performing undertaking, controlling or monitoring bodily functions. Retinal implants are a particular example. Such implantable electronic devices are typically in direct contact with bodily fluids and tissues. Therefore, electronic devices, and in particular implantable electronic devices for bio-medical *in vivo* applications should be packaged in order to 1) protect the implanted device against whatever impairment by the body's aqueous environment, such as the intrusion of redox-active or corrosive compounds into the device, which may cause corrosion, damage or improper function of the implanted device, and 2) protect the body against leakage of harmful products released from the electronic device into the body's tissues, or against other disadvantageous effects for the patient's body, such as mechanical friction by the implanted device in the body.

Prior art approaches for packaging implantable electronic devices typically place the device in a metal housing. In this known way of packaging, the package is much larger than the original chip, requiring larger incisions during implantation, thus resulting in a more extensive wound healing and inflammation processes. Furthermore, the larger the implant, the larger the fibrous encapsulation may be, resulting in a higher risk of local tissue irritation for the patient during the lifetime of the implant. The proposed hermetic packaging of the present invention is superior to said prior art approaches in that it is small, e.g. substantially of a similar size as or only slightly larger than the original device size itself. Its small size enables a less traumatic implantation and a faster wound healing. As compared to prior art methods of enclosing electronic devices each individually in metal housings, the inventive method offers the advantage of being applicable for simultaneous processing of multiple electronic devices or components of the same or different kinds, and is largely substantially independent of the substrate or component materials used. Moreover, the packaging can be performed in a clean environment, e.g. in clean room conditions after processing of the electronic devices or components, and in this way contamination and/or damage can be avoided. Furthermore, the inventive packaging method offers an exceptionally good step coverage resulting in overlapping layers and uniform thickness of the encapsulation, which provide a good hermetic seal.

US 2015/0297136 A1 describes an alternative method for packaging biomedical devices, which does not entail the inventive use of a removable layer as a temporary barrier for protecting the encapsulation layers from degradation during manufacturing. Due to its different method of processing, US 2015/0297136 A1 does not enable a hermetic encapsulation with only two layers and necessarily requires the use of an additional coating to achieve a hermetic seal. Specifically, the inventive packaging method enables the provision of a hermetically sealing double-layer covering the entire electronic device, and in particular its side walls. Such double-layered structures are particularly advantageous for ensuring a hermetic seal, as single layers may comprise pin holes (i.e., microscopic defects), which may constitute entry points for surrounding, potentially corrosive, media. A second layer of material will close these pin holes and secure the hermetic sealing of the encapsulation. With prior art methods, it was not possible to provide a double-layered seal fully surrounding all sides of an electronic device, mainly due to the fact that electronic device needs to be fastened during coating, rendering some of their surfaces inaccessible. It was not until the provision of the inventive packaging method, which involves flipping of the device and using a removable layer such as photoresist as a temporary protective barrier, that it became possible to deposit conformal sealing materials on the side walls from both sides and therefore provide a double layered fully surrounding seal. In addition, in contrast to the packaged device described in US 2015/0297136 A1, the inventive packaging method obviates the need for sloped side walls of the device, and can be used with highly conformal coatings that are capable of coating straight and right-angled surfaces as commonly present in biomedical implants. Furthermore, US 2015/0297136 A1 does not relate to retinal implants or envisage the use of a top coating, let alone a transparent top coating as described in the present invention.

It is an advantage of the inventive method and packaging that a reliable hermetic encapsulation with a reduced number of pinholes can be obtained with only two overlapping encapsulation layers and without the need for an additional embedding layer surrounding the packaged chip. US 2013/330498 A1 and US 2011/039050 A1 disclose an implantable medical device comprising an electronic component being encapsulated by a packaging with encapsulation layers stacked onto each other, thus forming a multi-layer encapsulation.

### SUMMARY

The invention is defined by the independent claims. Inventive aspects relate to methods for hermetically packaging electronic components for manufacturing packaged electronic devices, e.g. implanted electronic devices for bio- or bio-medical *in vivo* applications and packaged electronic devices obtained by such a method. Advantageously, the packaging according to the invention provides an improved hermetic barrier, which preferably minimizes the interaction between the electronic device or its functional electronic component on the one hand and its environment on the other hand, e.g. the *in vivo* environment when implanted. Thereby, the inventive packaging preferably 1) ensures maintenance of the function of the electronic device under in vivo conditions, e.g. by avoiding improper function of the device, e.g. due to corrosion or short circuit effects, and 2) protect the body against leakage/leaching or diffusion of materials of the device's electronic component into the surrounding tissue when implanted, or against other adverse effects, such as mechanical friction of the implanted device in the body. Put differently, the hermetic packaging characterizing the present invention's device establishes a reliable barrier against external factors. More specifically, the invention may provide a bi-directional diffusion barrier without affecting the device by its in vivo environment and without diffusion of unphysiological or non-biocompatible materials of the electronic component into the tissue surrounding the implanted device. The advantageous properties of the inventive packaging are preferably obtained by providing layered encapsulation fully encapsulating the electronic component. The resulting packaged device exhibits at least partially overlapping layers embedding the electronic component to form a double layer structure, which is preferably obtained or obtainable using the inventive packaging method.

In a first aspect, the invention relates to an implantable packaged device comprising an electronic component and a hermetic package encapsulating the electronic component, wherein said packaging comprises a top and a bottom encapsulation layer, which least partially overlap so as to form a double layer structure. Preferably, the double layer structure at least partially, more preferably fully, extends over and thereby covers the electronic component's side walls. In this way, the packaging of the device may advantageously provide a hermetic seal for its electronic component. As used herein, the terms "hermetic", "hermetically seal" and "hermetic seal" means impervious or essentially impervious to undesired external factors negatively affecting the device's function. That is, a "hermetic" seal or encapsulation preferably shields the implantable device from its environment, in particular the aqueous in vivo environment in an effective way, such that corrosion or whatever other functional impairment of the device is preferably minimized or avoided. Preferably, a "hermetic" seal or layer prevents the ingress of bodily fluids to the device. The term "hermetic" may further mean that the body is likewise shielded from the implanted device.

The packaged device is typically defined by side wall surfaces and by a top and bottom surface. The terms "top encapsulation layer" and "bottom encapsulation layer" are typically meant to cover - at least partially - the upper (top) surface of the electronic component to be encapsulated or the bottom surface of the electronic component. Thus, the "upper surface" of the electronic device is fully or partially covered by one or more layers, the outermost thereof defining the top surface of the packaged implantable device, whereas the bottom surface of the electronic component to be packaged is covered by one or more bottom layers, the outermost thereof defining the bottom surface of the packaged implantable device.

It is understood that the device may contain feedthroughs or holes for communication with its in vivo environment, e.g. by electrical stimulation of the surrounding cells or tissues. The "upper portion" of the packaged implantable device contains functional structures or features allowing the device to interact with its environment, e.g. by comprising stimulating or recording electrodes or photodiodes. The "top surface" of the implantable packaged device thereby (partially) covers its upper portion, whereas the "bottom surface" covers its lower portion. The top surface or a portion thereof may - for some embodiments - expose a coating layer as the outermost layer being in contact with the environment. In terms of the production method to provide a packaged device according to the invention, it is noted that the production method is characterized by initial steps applying one or more encapsulating layer/s on the top surface, while the bottom surface is only thereafter established by applying one or more bottom encapsulating layer/s.

In a second aspect, the invention provides an implantable system comprising the hermetically packaged device according to the invention.

In a third aspect, the invention relates to a packaging method for providing or manufacturing an implantable device, whereby the method comprises (a) providing at least one electronic component present on a substrate, (b) applying at least one top encapsulation layer to an electronic component, and (c) applying at least one bottom encapsulation layer to the electronic component, wherein the top and bottom encapsulation layer at least partially overlap so as to form a double layer structure. Advantageously, the inventive method may allow processing multiple electronic components, such as electronic dies, simultaneously to manufacture a multitude of packaged electronic devices, which results in a cost-effective and scalable production process, especially for chip fabrication.

In a further aspect, which may also be an embodiment of the second aspect, the invention relates a method for packaging an electronic component to provide a packaged implantable device, said method comprising the steps of: (i) providing an assembly of at least one, preferably a plurality of electronic components separated from each other on a substrate by introducing recesses into an electronic component proto-structure, wherein adjacent electronic components and their common substrate support or define the recesses; (ii) applying at least one top encapsulation layer to an assembly, thereby coating the electronic components and lining the recesses; (iii) applying a removable layer to the assembly; (iv) partially removing the removable layer, thereby retaining a residual amount of the removable layer within lined recesses; (v) optionally flipping the assembly upside down; (vi) removing a) substrate, b) top encapsulation layer and c) residual amount of removable layer from the assembly's bottom side; and (vii) applying at least one bottom encapsulation layer to the assembly, wherein top and bottom encapsulation layers are applied so as to at least partially overlap, thereby forming a double layer structure. According to the inventive method, the top or upper side of the assembly is initially processed by adding the top encapsulation layer and the removable layer. Subsequently, the assembly is preferably flipped upside down (thus allowing the bottom side to be processed preferably from below and, less preferably, from above) to remove the substrate from the assembly's bottom side, and the top encapsulation layer below the removable layer within the recesses. Thereby, a residual amount of the removable layer is retained in each recess, which preferably protects the top encapsulation layer lining the sidewalls of the recesses against degradation. Subsequently, the removable layer is typically completely removed. Finally, the bottom encapsulation layer is applied to the assembly's bottom side and the sidewalls. Advantageously, the inventive method - preferably by flipping the assembly upside down during processing and retaining the removable layer for protection - allows for a hermetic packaging based on stacked encapsulation layers extending over the electronic component's side walls, which enables improved hermetic properties.

The methods according to the third and fourth aspect of the invention for packaging an implantable device allow preferably to prepare a packaged device according to the first aspect of the present invention.

### BRIEF DESCRIPTION OF THE DRAWINGS

FIG. 1 A - F illustrates packaged devices 1 according to preferred embodiments of the invention.
FIG. 2 illustrates a method for producing or manufacturing an encapsulated packaged device comprising an electronic component characterized by steps 309 through to 317 according to a preferred embodiment of the present invention.
FIG. 3 illustrates another packaged device according to the invention embedding an electronic component by a hermetic packaging.
FIG. 4 A-C illustrate exemplary optional step 400 for applying top coating 107 according to three alternative embodiments.

### DETAILED DESCRIPTION

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the features of the present invention will be described. These features are described for specific embodiments. It should, however, be understood that they may be combined in any manner and in any number to generate additional embodiments. The variously described examples and preferred embodiments should not be construed to limit the present invention to only explicitly described embodiments. This present description should be understood to support and encompass embodiments, which combine the explicitly described embodiments with any number of the disclosed and/or preferred features. Furthermore, any permutations and combinations of all described features in this application shall be considered supported by the description of the present application, unless it is understood otherwise.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein. It is to be noticed that the method steps described herein may be interchanged in order as appropriate. In the following description, by way of illustration, a number of examples and embodiments with interchanged steps will be discussed, the present invention not being limited thereto.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" e.g., a composition which is "substantially free" from Y may be completely free from Y. Where necessary, the word "substantially" may be omitted from the definition of the invention.

The term "about" in relation to a numerical value x means x ± 10%.

In a first aspect, the present invention relates to a hermetically packaged electronic device comprising an electronic component. Preferably, said device is an implantable device. More preferably, said electronic device may be a retinal implant suitable or configured to be implanted in the eye. The electronic device according to the present invention comprises a hermetic packaging, which is characterized by a stacked or double layer structure, preferably extending over or at least partially covering the electronic component's side walls. In this way, the packaging ensures an improved hermetic encapsulation of the device for becoming suitable for long term implantation. Preferably, the hermetic packaging prevents or reduces adverse effects resulting from the aqueous in vivo environment after implantation, such as the intrusion of bodily fluids or cells, and diffusion of non-biocompatible agents, e.g. metals, from the electronic component into the body's in vivo environment. Preferably, the packaged device is enclosed or embedded by at least two corrosion-resistant encapsulation layers, i.e. a top and bottom encapsulation layer. The hermetically packaged device may also have more than two encapsulation layers. For instance, the device may comprise one or more top encapsulation layers that are layered on top of each other, and/or one or more bottom encapsulation layers that are partially of fully layered on top of each other. The outermost layer(s) may preferably be biocompatible. The hermetically packaged device may include further coatings, such as a top coating introducing further properties into the claimed electronic device. E.g., in case of hermetically packaged photovoltaic (retinal) implants, the top coating and/or top encapsulation layer may be made of transparent material for receiving data by encoded by light signals, e.g. visible or IR light. In case of an electronic device according to the invention serving as an implantable stimulator or an implantable recording device, the top coating or top encapsulation layer may embed electrodes. Thus, the electrodes and/or the photodiodes, preferably positioned at the top side of the electronic device, are preferably exposed to the environment such that at least their outer top surface is not covered by any encapsulation layer. More preferably, the electrodes and/or photodiodes are embedded by at least one encapsulation layer, in particular by a top coating and(or top encapsulation layer. The top and bottom encapsulation layers may be composed of one or more conductive (sub)-layers, which may e.g. be patterned for instance as electrical tracks, thus allowing e.g. for one or multiple electrical connections between the top and bottom side of the devices.

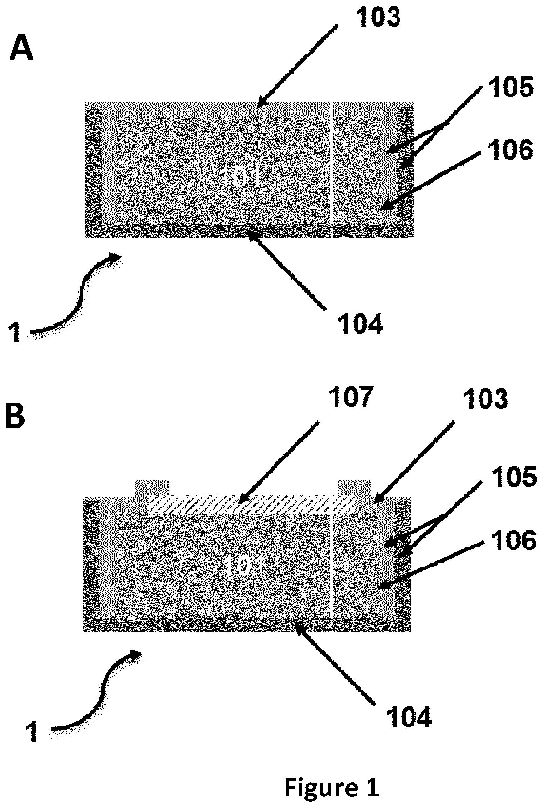

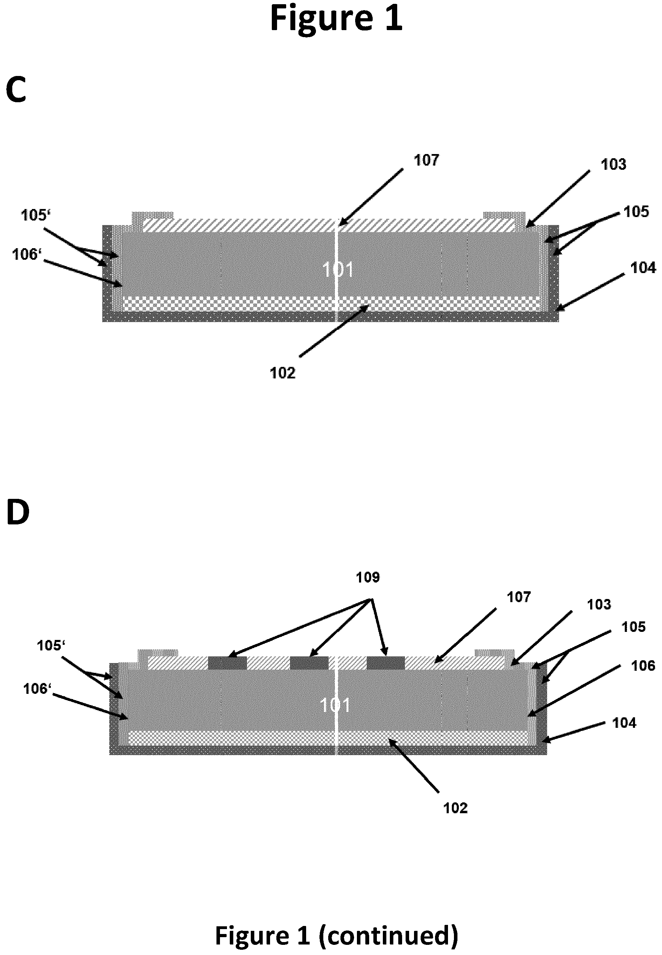

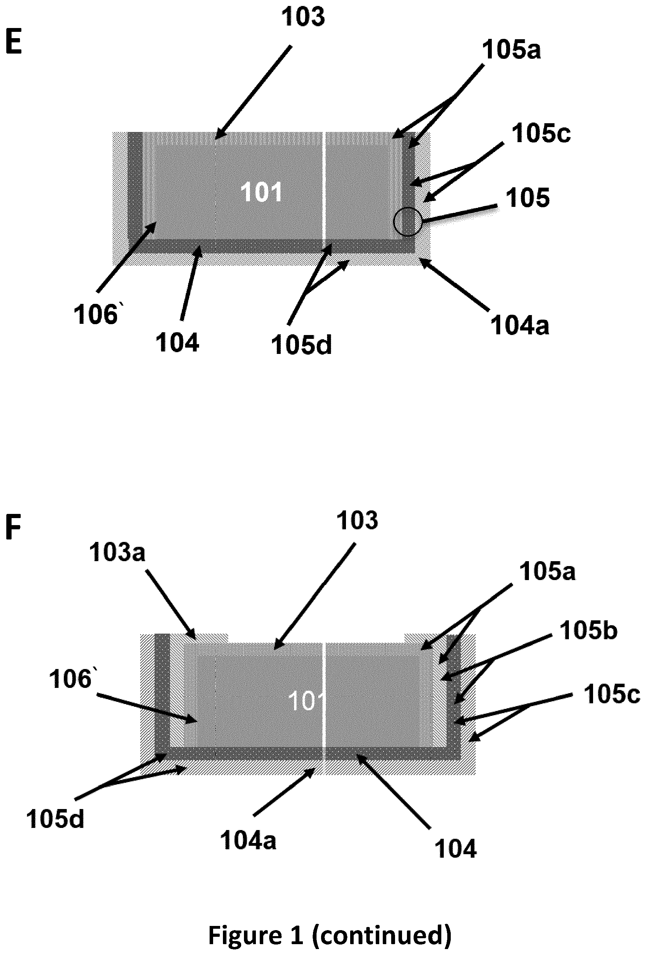

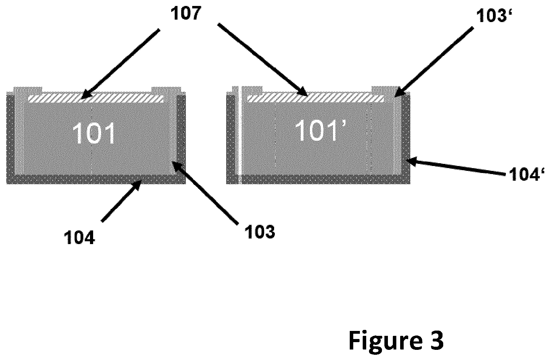

By way of illustration, and without being limited thereto, exemplary packaged implantable devices **1** are shown in **FIG. 1** **A - F** and **FIG. 3****.** The packaged electronic device according to the invention comprises an electronic component **101** encapsulated by a hermetic packaging (whereby the hermetic packaging is defined by the layers surrounding and encapsulating electronic component **101**), said packaging comprising at least top encapsulation layer **103** and a bottom encapsulation layer **104.** The top and bottom encapsulation layers **103, 104** at least partially overlap so as to form areas of a double layer structure/s **105, 105',** e.g. at the side walls of the encapsulated device. The top and bottom encapsulation layer are thus typically designed to not only (at least partially) cover the top surface and the bottom surface, respectively, of the electronic component to be packaged, but also extend beyond the top or the bottom surface, e.g. in vertical direction, thus also covering (at least partially) the side walls of the device. For the embodiments exemplified in **FIG. 1** **A** to **D,** bottom encapsulation layer **104** forms the outer layer of the encapsulated device at the areas of double layer structure **105, 105'.** However, an assembly **100** with the top encapsulation layer **103** forming the outermost layer of the device at such areas of double layer structure **105, 105'** is disclosed herewith as well.

The packaged electronic device **1** may generally be an electronic device of any sort, including a chip, stimulator, and a control or monitoring device. The electronic device is preferably implantable. The packaged electronic device is preferably useful for biological or biomedical applications, in particular for *in vivo* applications. Exemplary implantable electronic devices include retinal implants, including sub- and epiretinal implants, e.g. those described in WO2016/180517 and PCT/EP2018/069159, or brain implants, e.g. for stimulating the visual cortex. Such implants allow to e.g. electrically stimulate neurons, e.g. within a patient's eye or brain (e.g. for regaining visual perception by stimulating the visual cortex or by stimulating neurons for treating Parkinson's disease), and/or to record electrical signals of the patients neurons. The hermetic packaging described herein ensures long term implant half life without the implanted electronic device being affected by the aqueous in vivo environment at the implantation site.

The electronic device **1** according to the present invention may include one or more electronic components **101,** which is/are advantageously of cuboid shape and does/do preferably not comprise any electronic elements, e.g. a transistor, of three-dimensional shape projecting outwardly (projecting out of cuboid or the planes of the cuboid), to be packaged, which may be integrated circuits, also referred to as dies. Further types of such components include micro electromechanical systems (MEMS), including 0-th level packaged MEMS, thin-film capped MEMS, etc. The MEMs devices include for instance passive components, actuators, sensors, etc. Further examples of such components include micro-fluidics devices. Still further examples include batteries, such as a rechargeable battery, circuits, transistors, resistors, photodiodes, capacitors and the like. Electronic components **101** may actually represent a stack of more than one individual electronic units, for instance memory chips on integrated circuits. Stacked units forming an electronic component **101** may be encapsulated as whole such that packaged implantable electronic device **1** comprises electronic component **101** comprising a stack of electronic units.

Electronic components **101** may - in addition to the electronic units - also comprise a (non-encapsulating) layer **102.** It may be positioned at the bottom surface of the electronic component **101** (see **FIG. 1** **C** and **D**), but alternatively also at its top surface. Layer **102** may comprise or consist of any suitable substrate material including, for example, a semiconductor material, e.g. a silicon material, an electrically insulating material, a glass material, a polymer material, and if suitable an electrically conducting material such as for example a metal. Preferably, layer **102** may comprise or consist of ceramics or glass, optionally selected from silicon oxide or dioxide, optionally obtained by oxidation of a silicon substrate. Layer **102** may advantageously act as a barrier protecting the electronic component **101** in the course of the packaging method, in particular at the step of removing substrate **110.** For instance, layer **102** may be composed of a material that is not prone to decomposition/degradation when subjected to the step of removal of substrate **110.** Electronic component **101,** layer **102** and substrate **110** may preferably be selected to form a "silicon on insulator" or "SOI" wafer structure, which is readily available and widely used in the art. To this end, layer **102** may preferably be configured as a layer of silicon oxide disposed on a silicon substrate **110.** Specifically for photodiodes or other light sensitive electronic components **101,** the silicon dioxide layer **102** may preferably be thermally grown on electronic component **101** using known methods in the art, in order to provide an interface between electronic units/s and layer **102** of component **101** with favourable electronic and/or optical properties.

It is preferred that double layer structure 105, 105' resulting from top and bottom encapsulation layers 103, 104 at least partially, more preferably fully, covers side walls 106, 106' of electronic component 101. The double-layered structure covering or at least partially extending over side walls 106, 106' advantageously confers an improved hermetic encapsulation and may, in some embodiments, preferably obviate the need for an additional encapsulation layer surrounding top and bottom encapsulation layer 103, 104, respectively. The double layer (105, 105') does preferably not fully, but only partially cover the electronic component (101), e.g. the double layer (105, 105') covers the side walls (106, 106') of the electronic component (101) only, but neither the top side nor the bottom side of electronic component (101) are covered by double layer (105, 105', 105a, 105b, 105c, 105d). Alternatively, the double layer (105, 105') may cover the side walls (106, 106') and the bottom side of the electronic component (101), but not the top side exposing photodiodes and/or electrodes (109). In another embodiment, only the bottom side of the electronic component (101) is covered by a double layer (105, 105', 105a, 105b, 105c, 105d).

Top and/or bottom encapsulation layer/s **103, 104** are preferably capable of providing a hermetic seal for electronic component **101.** The terms "hermetic seal" and "hermetically sealing" and "hermetic" are defined elsewhere herein. Top and/or bottom encapsulation layer **103, 104** may be advantageously biocompatible. The term "bio-compatible" and "bio-compatibility" refer to the ability of a medical device to perform its intended function in the host without eliciting any undesired local or systemic effects for the host. Top and bottom encapsulation layer **103, 104** may be corrosion-resistant. The term "corrosion resistant" refers to the material's resistance against its reaction with its aqueous environment as typically experienced under in vivo conditions. Redox-active compounds may corrode the device's material. Corrosion of an implant in vivo means that the implant is typically oxidized or otherwise chemically attacked by its environment. "Corrosion resistance" is the capacity to withstand deterioration and chemical modification/degradation, e.g. by redox reactions.

For embodiments (see e.g. embodiments of **FIG. 1** **A** or of **FIG. 1** **B** to **D** and of **FIG. 3** with an additional coating of the top surface) characterized by an hermetic packaging which is composed of one single top and one single bottom encapsulation layer **103, 104** only overlapping with each other to preferably at least partially cover the electronic component's side walls **106, 106',** i.e. the hermetic packaging does not comprise any further encapsulation layer, it is preferred that top and bottom encapsulation layer **103, 104** are both biocompatible and corrosion-resistant, in order to ensure both in vivo compatibility and proper function of the electronic device **1.** The skilled person is readily able to select suitable materials for the top and bottom encapsulation layer **103, 104** preferably exhibiting bio-compatibility and/or corrosion-resistance. Exemplary suitable materials for the top and bottom encapsulation layer **103, 104** include metals; ceramics including oxides, nitrides, and carbides; diamond-like carbon; diamond; glass; polymers, in particular low permeability and/or dense polymers; and combinations thereof. Specifically, suitable metals may be selected from titanium (Ti), platinum (Pt), stainless steel, titanium-nickel, palladium, niobium, tantalum, and combinations or alloys thereof; suitable ceramics may be selected from silicon oxide, silicon nitride, silicon carbide, silicon oxycarbide, titanium carbide, titanium nitride, titanium oxide, aluminum oxide, aluminum nitride, zirconium oxide, and combinations thereof; suitable polymers may be selected from fluorocarbons, polyurethane, polyether ether ketone (PEEK), silicone, PDMS, parylene, polyimide, polycarbonate, polycarbonate urethane, silicone, silicone-polyester-urethane, durimide (photo-definable polyimide), cyclic olefin polymer (COP), cyclic olefin copolymer (COC), polymethyl methacrylate (PMMA), polyphenylene, polysulfone, polyphenylsulfone, combinations thereof and multilayers thereof. Top and bottom encapsulation layer **103, 104** may comprise or consist of the same or different materials. Top and bottom encapsulation layer **103, 104** may comprise or consist of monolayers or multilayers (e.g. more than one (sub)-layer) of the materials specified herein.

As indicated, hermetic packaging may comprise at least one additional top encapsulation layer **103a,** and/or an additional bottom encapsulation layer **104a** (see e.g. embodiments of **Figures 1** **E** and **1 F**), which preferably form at least one additional double layer structure **105a, 105c** at the side walls and at least one additional double layer structure **105d** at the bottom (**FIG. 1** **E**) or at least two additional double layer structures (**105a, 105b and 105c**) at the side walls (**FIG. 1** **F**). Such an additional double layer structure **105b, 105c** may be due to the outer encapsulation layers **104** and **104a** at the bottom side and/or the side walls of the device. In particular, such an additional bottom encapsulation layer **104a** may cover at least partially, more preferably fully, side walls **106, 106'** of electronic component **101.** It may result - as shown for the embodiments of **FIG. 1** **E** and 1 **F** - in a triple encapsulation layer side wall arrangement with the top encapsulation layer **103** forming an inner double layer structure **105** together with bottom encapsulation layer **104.** The bottom encapsulation layer **104** furthermore forms a second double layer structure **105c** together with the additional bottom encapsulation layer **104a** resulting in a triple layer structure at the side walls. Preferably, the at least one additional double layer (105a, 105b, 105c) covers the side walls (106, 106') only, but neither the top side nor the bottom side of the electronic component (101). Analogous arrangements may be provided with the bottom layer encapsulation **104** being the innermost encapsulation layer at the side wall areas such that the top encapsulation layer **103** is sandwiched between the additional bottom encapsulation layer **104a** as the outermost layer and the bottom encapsulation layer **104** being in such an embodiment the innermost encapsulation layer at the side wall areas. Moreover, the additional bottom encapsulation layer **104a** may establish another double layer structure **105d** at the bottom side of the device with bottom encapsulation layer **104.** **FIG. 1** **F** exemplifies the embodiment of **FIG. 1** **E,** however, additionally containing a partial additional top encapsulation layer **103a** on top of encapsulation layer **103,** thereby forming a double layer structure at a peripheral region of the top side of the packaged electronic device **1** according to the invention as well. Three double layer structures **105a, 105b and 105c** (four side wall layers) are provided at the side walls of device **101.** The use of more than one or more than 2 stacked encapsulation layers may advantageously improve hermetic encapsulation and provide an even more reliable barrier against external influences. Embodiments using such multilayer layer configurations are still compatible with undelayed wound healing and low local tissue irritation risk upon implantation surgery, as the additional layer does not prominently enlarge the implanted device.

To this end, hermetic packaging may be provided with at least one additional top encapsulation layer **103a** and/or at least one additional bottom encapsulation layer **104a.** Additional top encapsulation layer **103a** is preferably applied onto encapsulation layer **103.** Additional bottom encapsulation layer **104a** is preferably applied onto bottom encapsulation layer **104.** Thereby, additional top and bottom encapsulation layer **103a, 104a** preferably form the outermost layers of hermetic packaging and are thus in direct contact with the environment, i.e. the body tissue, when implanted. Top and bottom encapsulation layers **103, 104** are sandwiched between the outermost layers **103a, 104a** and the top and bottom surface of electronic component **101.** Therefore, top and bottom encapsulation layers **103, 104** may, but need not necessarily, be composed of a biocompatible material (in particular whenever they are not in direct contact with body tissue). However, top and bottom encapsulation layers **103, 104** preferably comprise or consist of a corrosion-resistant material as specified above in order to protect electronic component **101** against corrosive degradation by the environment. Additional top and/or bottom encapsulation layers **103a, 104a,** if present, may, but need not necessarily be composed of a corrosion-resistant material (in particular, whenever layers **103, 104** are suitable to protect electronic component **101** against corrosion). Layers **103a** and **104a,** if present, are preferably composed of a biocompatible material.

Additional top and bottom encapsulation layers **103a, 104a** may be composed of monolayers or multilayers (e.g. more than one (sub)-layer). Additional top and bottom encapsulation layers **103a, 104a** may be composed of the same or different materials.

For instance, preferred embodiments exhibiting "stacked" or multiple encapsulation layers may include a top and a bottom encapsulation layer **103, 104** made from a corrosion-resistant material, such as a metal, and may further be embedded in additional top and/or bottom encapsulation layers **103a, 104a** made from a biocompatible material, e.g. silicone, parylene, hydrogels, etc.

(Additional) top and bottom encapsulation layers **103, 103a, 104** and **104a,** in particular in view of their optional multi-layered structure, may also comprise at least one conductive (sub)-layer that permits electrical connection between the top and the bottom side or portion of the packaged device. It may be preferred that the circuitry is positioned on the top side. One or several of those (sub)-layers can be patterned, for instance in the shape of electrical tracks, to permit one or more electrical connections between the top and the bottom side of the packaged device. For instance, electronic component **101** may have circuitry on its top and/or bottom side that are interconnected by one of several layers of electrical tracks that are part of at least one of the encapsulation layers. Encapsulation layers **103, 103a, 104, 104a** may therefore comprise at least one of patterned or non-patterned, electrically conductive or electrically isolating (sub)-layers, e.g. platinum, titanium, silicon carbide, silicon oxide, or silicon nitride. In one embodiment, the conductive encapsulation (sub)-layers, such as metals, may all be electrically connected to each other and the electrical ground of the circuitry of the electronic component 101. In another embodiment, the (patterned) passive conductive tracks or full conductive (sub)-layers of at least one of encapsulation layers **103, 103a, 104, 104a** serve to establish an electrical contact to the circuitry.

The electronic device **1** according to the present invention may further include at least one top coating **107** as illustrated in **FIG. 1** **B** - **D.** Top coating **107** is preferably at least partially overlapped by top encapsulation layer **103** to advantageously ensure hermeticity of the packaging. As top coating **107** typically forms an outermost layer of the hermetic packaging according to the invention, e.g. at the top side of the inventive device, it is in direct contact with body tissue when implanted and thus preferably biocompatible. Preferably, top coating 107 may be corrosion-resistant as well. Top coating **107** may be composed of any suitable material which preferably adds a further functionality to the hermetic packaging of the inventive device. E.g., in particular for retinal implants as implantable electronic devices **1,** top coating **107** may be composed of light transparent material. Advantageously, light (e.g. IR or visible light) transparent top coatings are useful for retinal stimulators, e.g., photovoltaic retinal stimulators. Specifically, top coating **107** may comprise or consist of a material selected from ceramic, including SiC, SiOC; SiO₂; glass; diamond or diamond like carbon; aluminum oxides; titanium oxides; and combinations thereof. Top coating **107** may be composed of monolayers or multilayers (representing (sub)-layers) of the above-specified materials. Each (sub-) layer may be composed of identical or preferably distinct materials, in particular, as disclosed above. The above-specified materials of top coating **107** may be provided in amorphous or crystalline form, or both.

As indicated, implantable electronic device **1** may preferably be an electrically stimulating device, such as a retinal implant or retinal stimulator. To this end, hermetic packaging may, e.g. by its top coating **107** and/or its top encapsulation layer **103,** comprise electronic traces **108** and/or cover, surround or embed electrodes **109** being part of and electronically connected to the electronic component **101.** Preferably, electrodes **109** are provided within or extending beyond the hermetic packaging, in particular within or projecting beyond top coating 107. Such an embodiment is illustrated in **FIG. 1** **D.** Top coating **107** may include holes or feedthroughs to pass or exchange electrical, light or chemical signals (e.g. for data transmission) to and from component **101.** Such feedthroughs may for example include electrodes **109** to transfer electrical or ionic signals. Other exemplary feedthroughs that may be provided may be liquid feedthroughs. Feedthroughs may be connected to electrical leads for receiving or transmitting electrical signals (e.g. to sense signals or to electrically stimulate target cells or tissues). Feedthroughs may be connected to flexible circuits connected to or in communication with further electrodes or devices remote from component **101.**

Preferably, the electronic component **101** is an integrated circuit or a die, with electrodes 109 being provided as feedthroughs. Electrodes **109** are preferably in electrical communication with electronic traces **108** disposed in or on said integrated circuit and may, e.g., transduce electrical signals generated through said integrated circuit. However, the skilled person readily understands that the provision of electronic traces **108** and/or electrodes **109** is not restricted to electrical stimulators, or retinal stimulators. Electronic traces may, for instance, also be provided in devices which are used as sensors or controllers or for other applications. In implanted devices 1, selected electronic traces **108** and/or electrodes **109** are preferably in direct contact with the body tissue when implanted. To this end, electrodes **109** may preferably be made of biocompatible material. Preferably, electronic traces **108** and/or electrodes **109** may be made of corrosion-resistant material to reduce or avoid corrosion and damage to electronic component **101.** Electronic traces **108** and/or electrodes **109** may preferably comprise or consist of a material selected from platinum, black/porous platinum, iridium, iridium/platinum, iridium oxide, poly(3,4-ethylenedioxythiophene) polystyrene sulfonate (PEDOT:PSS), PEDOT:PSS, (porous) titanium nitride, doped diamond or doped diamond like carbon, and graphene.

In some preferred embodiments, the inventive device **1** may be as illustrated by **FIG. 1** **C or D,** comprising at least one electronic component **101,** which preferably includes an integrated circuit or die and, optionally, includes further electronic units, such as passives or active circuits, fully packaged or embedded by a hermetic packaging comprising a top encapsulation layer **103** and a bottom encapsulation layer **104,** each composed of a metal, preferably titanium. Electronic components **101, 101'** may include a layer **102,** typically positioned at the bottom side of the electronic component, which may be composed of ceramics or glass, metal, or combinations thereof, preferably of silicon dioxide. In specific embodiments, layer **102** is foreseen as a double layer structure e.g. composed of one layer of ceramics or glass and one layer of metal, or e.g. of two metal layers. Electrodes **109** as part of component **101, 101'** are preferably made of platinum (Pt), including porous and platinum black, (porous) TiN, iridium oxide, or poly(3,4-ethylenedioxythiophene) polystyrene sulfonate (PEDOT:PSS), establishing an electrical connection between electronic component **101** and the surrounding bodily fluid, tissue or cells. Electrodes **109,** e.g. protruding upwardly such that they are embedded within top layer **107** or projecting beyond top layer **107,** are preferably configured to establish an electrical connection between component **101, 101'** and the surrounding environment, e.g. the body tissue when implanted. In preferred embodiments, electronic traces **108** may be provided as part of the electronic component **101, 101',** e.g. on the top or upper surface of components **101, 101'.**

The hermetic packaging of the device **1** preferably comprises a top layer **107,** which is preferably transparent. Top layer **107** may preferably be composed of a ceramic layer, more preferably a ceramic multilayer, even more preferably a ceramic multilayer comprising or consisting of silicon carbide, for instance amorphous silicon carbide. Top layer **107** may also comprise electronic traces, preferably from titanium (Ti), gold (Au), platinum (Pt), copper (Cu), palladium (Pd) or aluminum (Al) or a multilayer thereof,

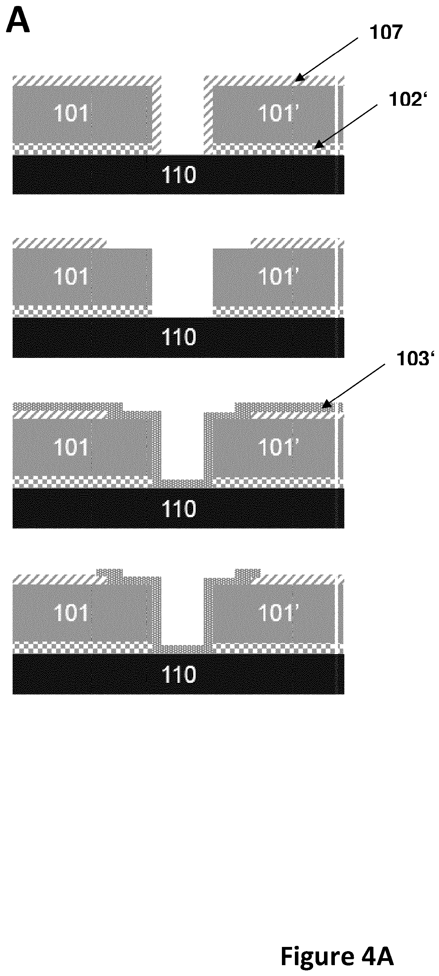

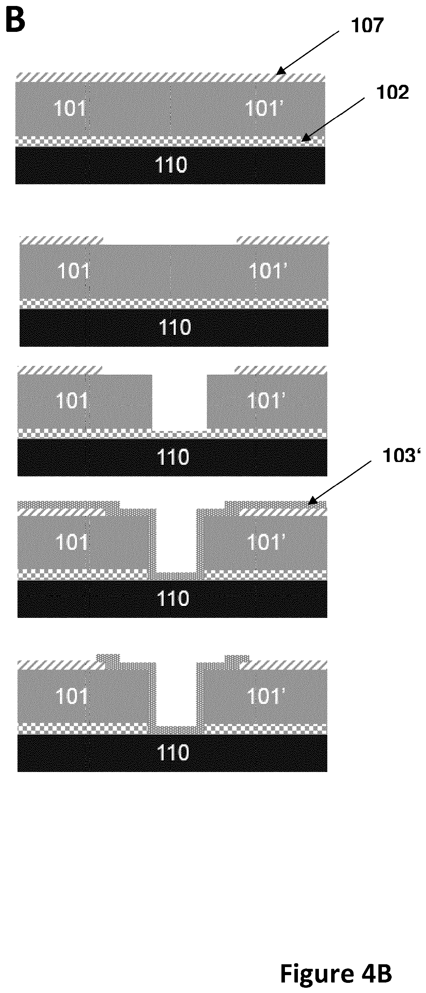

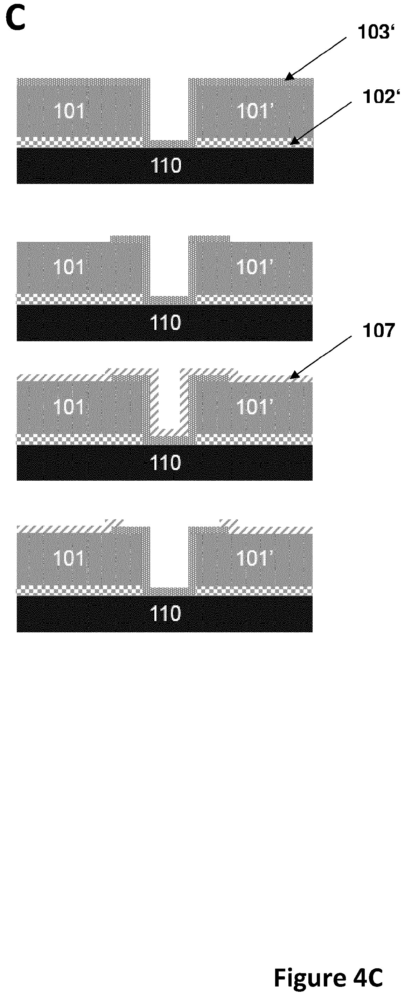

**Figure 3** shows another embodiment of an inventive packaged implantable device 1. The device according to **Figure 3** comprises the additional coating layer **107.** The top encapsulation layer **103** overlaps the coating layer **107** at its edge zone. Such a device 1 may e.g. obtained by carrying out the inventive method in combination with the steps of e.g. **Figure 4** **A** (step **300**).

Preferably, device **1** may be a retinal implant packaged by the hermetic packaging for implantation in the eyes, preferably configured for being implanted epi- or subretinally. It may alternatively be configured for being implantable in the neural cortex, in particular for contacting neuronal cortex cells involved in visual data processing.

In a second aspect, the invention provides a system, comprising at least one packaged implantable device **1** as described herein, e.g. suitable or configured for being implanted in the eye. Device **1** may be obtainable by a method for manufacturing the claimed device **1** as disclosed herein. The system may also comprise other implantable or external (non-implantable) devices or components. The inventive system may also comprise more than one, e.g. a plurality of implantable devices **1.** As indicated above, electronic components **101** to be packaged may include integrated circuits or dies, CMOS logics (such as steering, programmable devices), MEMS (such as membranes for example for pressure sensing, drug reservoirs, microfluidics for drug delivery), batteries, antenna (for example for loading a rechargeable battery, for programming a reprogrammable device).

The system may be provided together with external devices, such as video glasses and an external pocket processor in communication with component **101.** Typically, component **101** may communicate with external devices via wireless communication, e.g. through infrared communication, patterned light, RF communication, or any other suitable means. Alternatively, component **101** may communicate with external devices via wired connections, e.g. including intracutaneous wires. In preferred embodiments, the implantable component of the system according to the invention may comprise a hermetically packaged component **101** as described herein as device **1** and as illustrated e.g. in **Fig. 1** **D** as a retinal implant. Packaged component **101** may preferably be implanted into the eye, more preferably being suitable or configured for implantation epi- or subretinally. Packaged electronic components **101, 101'** may be stacked and/or may be spaced apart from each other on a suitable support, or both. Functionally distinct packaged electronic components **101, 101'** may be electrically connected via electronic traces, e.g. via metallization on a common support. The support may be flexible or stretchable. Packaged electronic components **101, 101'** may be bonded to the support via suitable bonding means. The system may be provided with a global feedthrough, e.g. an electrical feedthrough, a fluid feedthrough etc.

In a third aspect, the present invention provides a method for producing or manufacturing an implantable packaged device **1,** the method comprising (a) providing at least one electronic component **101** positioned on a substrate **110,** (b) applying at least one top encapsulation layer **103, 103a** to electronic component **101,** and (c) applying at least one bottom encapsulation layer **104, 104a** to electronic component **101,** wherein the top and bottom encapsulation layer **103, 103a, 104, 104a** at least partially overlap so as to form a double layer structure **105, 105', 105a, 105b, 105c, 105d,** e.g. a double layer structure at/on the side wall areas of device **1,** at/on the bottom side of device **1** and/or at/on the top side device **1.** The double layer (105, 105', 105a, 105b, 105c, 105d) does preferably not fully, but only partially cover the electronic component (101), e.g. the double layer (105, 105') covers the side walls (106, 106') of the electronic component (101), but neither the top side nor the bottom side of electronic component (101) are covered by double layer (105, 105', 105a, 105b, 105c, 105d). Alternatively, the double layer (105, 105') may cover the side walls (106, 106') and the bottom side of the electronic component (101), but not the top side exposing photodiodes and/or electrodes (**109**). In another embodiment, only the bottom side of the electronic component (101) is covered by a double layer (105, 105', 105a, 105b, 105c, 105d).

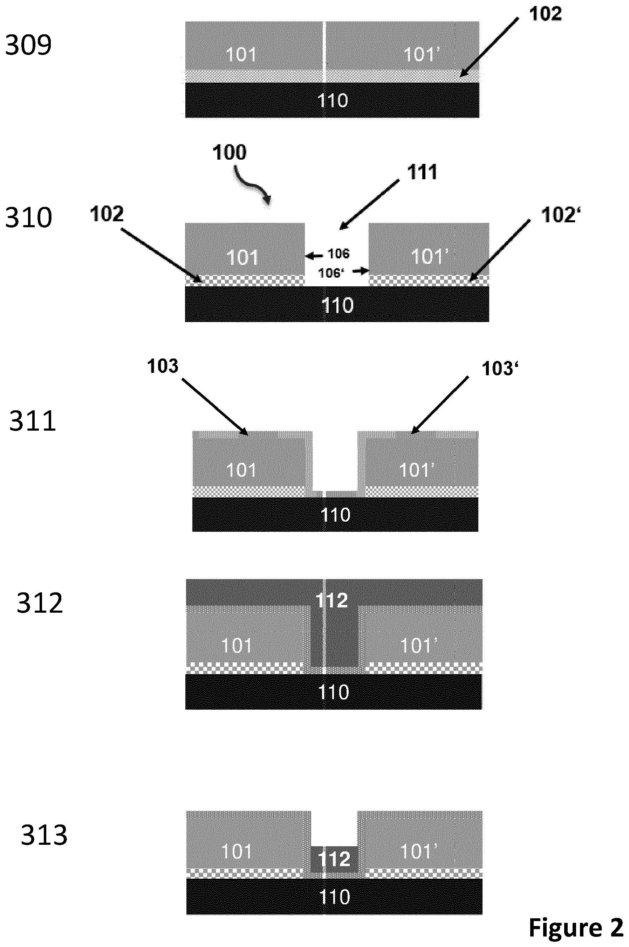

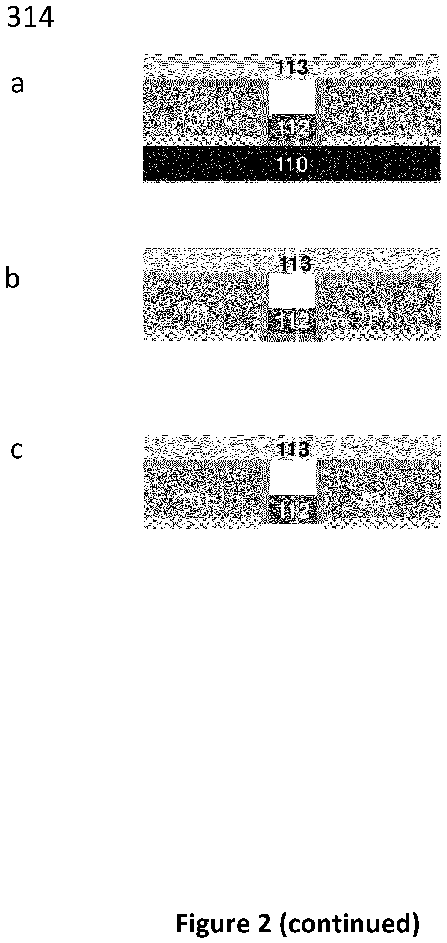

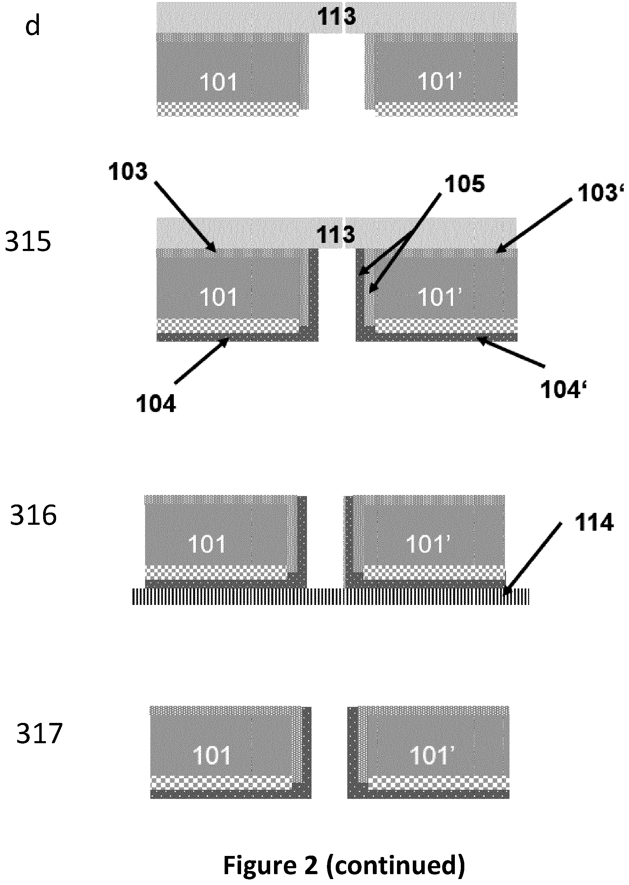

In a fourth aspect, which may be an embodiment of the third aspect, the present invention provides a method (as e.g. illustrated in **FIG. 2**) for producing or manufacturing an implantable packaged device, said method comprising the following steps **309, 310, 311, 312, 313, 314, 315,** optional step **316,** and **317,** preferably according to that order of steps.

In step **309,** an assembly **100** is provided as a proto-structure with a continuous layer **102** and a continuous electronic component proto-structure **101** being positioned thereon. At its bottom, assembly **100** is supported by substrate **110.**

In step **310,** an assembly **100** of at least one, preferably a plurality of electronic components **101** and **101'** is provided, with said electronic components **101** and **101'** being spaced apart and separated from each other on substrate **110** by a recess or gap. Adjacent electronic components **101, 101'** and substrate **110** define recesses **111.** The recesses **111** are laterally surrounded by side walls **106, 106'** of electronic components **101, 101'.** In the present example, the assembly may be an assembly of dies **101, 101'** on a wafer **110.** Advantageously, substrate **110** may support a larger plurality of e.g. at least 10 or at least 20 or at least 50 or at least 100 or at least 500 components **101, 101'.** The provision of assembly **100** according to step **310** includes introduction of recesses **111** into at least one continuous electronic component proto-structure **101** (as shown by step **309** of **Figure 2**) serving as the basic structure for the electronic components on which the inventive method is applied. It is composed of suitable materials (as described in the context of component **101** and layer **102**), with potentially several electronic and/or optical features, such as electronic circuitry or photodiodes or other sensor/stimulator features, and being typically made of several patterned (sub)-layers, disposed on/in substrate **110.** By step **310,** electronic components **101, 101'** are separated from each other by introduction of recesses **111** and thus singulated. Recesses **111** separating electronic components **101, 101'** are introduced e.g. by dry or wet etching. That step of separating the electronic components from each another permits the manufacture of a larger number of electronic components on the same (typically flat) substrate **110** based on one single electronic component proto-structure. This may allow the inventive packaging method to be applied simultaneously to a plurality of identical or distinct (depending on the nature of the proto-structure) electronic components **101, 101',** which enables a time- and cost-efficient manufacturing process.

In step **311,** at least one top encapsulation layer **103** is applied to the top surface of assembly **100,** thereby coating the upper surface of electronic components **101, 101'** horizontally and lining the walls of recesses **111** vertically. Suitable materials for top encapsulation layer **103** are described above. It will be understood that top encapsulation layers **103, 103a** may preferably be composed of a conformal material, which is capable of conforming to the contours of electronic components **101, 101'** and recesses **111** and thereby covers components **101, 101'** and the walls of recesses **111.** Due to the preferred choice of conformal materials and deposition techniques, such as non-directional or partially directional physical vapor deposition techniques, sputtering, or chemical vapor deposition, for top encapsulation layer **103,** a reliable and reproducible step-like (non-flat) coverage (including horizontal surface and vertical side wall coverage) is typically achieved. As indicated, step **311** may also comprise adding monolayers or multilayers of the same or different materials. Step **311** may also comprise applying at least one additional top encapsulation layer **103a** (not shown in Figure 2, step 311) to assembly **100,** typically after applying top encapsulation layer **103.** At least one of the (additional) top encapsulation layers **103, 103a** is preferably selected from a corrosion-resistant material sufficient to hermetically seal component **101** against the environment. The outermost top encapsulation layer 103 of the packaging, or, when adding an additional top encapsulation layer, outermost encapsulation layer **103**a is preferably composed of a biocompatible material in order to reduce or avoid irritation or damage of the surrounding body cells, tissues or fluids.

In step **312,** a removable layer **112** is applied to assembly **100,** typically on its upper surface, to cover the previously coated components **101, 101'** and lined recesses **111.** Preferably, removable layer **112** may comprise or consist polymeric material preferably selected from a resin, more preferably a photosensitive resin (photoresist), and a dissolvable polymeric material. Photosensitive resins or photoresists (also known as photopolymers or light-activated resins) are oligomers or polymers that alter their properties when exposed to light, typically in the ultraviolet or visible region of the electromagnetic spectrum. Specifically, upon irradiation of light, photosensitive resins either polymerize into insoluble cross-linked network polymers ("negative photoresist") or decompose solid polymers into semi liquid or soluble or dissolvable ("positive photoresist"). In the context of the present invention, positive photosensitive resins as commonly known are used. Removable layer **112** is applied as a temporary protection of components **101, 101'** and, more specifically, top encapsulation layer **103, 103a** for subsequent processing. Therefore, removable layer **112** is preferably composed of a material that can be removed without affecting electronic components **101, 101'** or top encapsulation layer **103, 103a.** Removing removable layer **112** may preferably be accomplished by applying photoresist developers or photoresist strippers as commonly used in the semiconductor industry.

In step **313,** removable layer **112** is removed from the assembly to expose top encapsulation layer **103** on the surface of components **101, 101'.** The removing step may be accomplished by any suitable physical and/or chemical means, including grinding, dry or wet etching and/or stripping in wet solutions or in a plasma. Preferably, removing may preferably leave a residual amount of removable layer **112** in lined recesses typically covering the bottom surface of the recess. This residual amount of removable layer **112** is intended to form a protective barrier or "plug" protecting the top encapsulation layer lining recesses **111** against degradation by subsequent processing steps.

In particular in preferred embodiments with removable layer **112** being composed of a positive photoresist, step **313** may include sub-step 1) exposing assembly **100** to light that is directed only to the top surface (but not to the bottom portion of recesses **111**) and sub-step 2) applying a photoresist developer to assembly **100.** In this way, it is ensured that only the photoresist removable layer **112** at or near the top surface of assembly **100** is removed, while a residual amount of unexposed photoresist remains within the bottom portion of recesses **111.** Alternatively, removable layer **112** may be composed of a negative photoresist. Under such circumstances, step **313** may include sub-step 1) exposing assembly **100** to light that is directed only to the bottom portion of recesses **111** (but not to the top surface) and sub-step 2) applying a photoresist developer to assembly **100.** In this way, it is ensured that only the unexposed photoresist removable layer **112** at the top surface of assembly **100** is removed, while a residual amount of photoresist remains within the bottom portion of recesses **111.**

In step **314** (as shown in **FIG. 2****,** step 314a), assembly **100** is flipped (not shown) and processed upside down. The flipping of the device facilitates the processing of its bottom side by making the bottom side accessible from above. To this end, assembly **100** may be temporarily attached to temporary carrier **113.** Temporary attachment may be accomplished by any suitable adhesion or bonding means, which are preferably reversible, e.g. via a suitable adhesive. Preferably, the adhesive may be characterized by an adjustable adhesive connection that can be modified, e.g. reduced by the application of e.g. heat, UV irradiation, laser light or other light irradiation so as to induce debonding of assembly **100** from temporary carrier **113.** Temporary carrier **113** may be composed of any suitable solid material, e.g. silicon or glass.

This processing advantageously allows for the provision of stacked or double-layered encapsulation layer structures **105, 105',** which ensures a hermetic sealing of the electronic component **101, 101'** by the resulting packaging.

Step **314** includes several sub-steps of removing the layers from the bottom of assembly **100** such that lined recesses **111** are left open for being finally lined with bottom encapsulation layer **104, 104'** (see following step **315**) to form a double layer structure **105, 105'** at least partially, more preferably fully, covering the electronic components' side walls **106, 106'.**

In step **314 a,** substrate **110** is removed, thereby exposing the electronic components' **101, 101'** bottom side, optionally covered by layer **102,** and the top encapsulation layer lining recesses **111.** Step **314a** may also be referred to as "thinning" of the substrate. Substrate **110** may be removed by any suitable physical and chemical means, including grinding and etching. Preferably, optional layer **102** may act as a barrier protecting the components' **101, 101'** bottom side against thinning or from whatever other damage resulting from the removal step, and may thereby allow precise control of the removal of substrate **110** without affecting electronic components **101, 101'.** The "thinning" is preferably performed until substrate **110** has been removed (step **314b**) or, more preferably until the residual amount of removable layer **112** left within recesses **111** is exposed by removal of layer **103, 103'** (sub-step **314 c**). In this way, electronic components **101, 101'** remain connected only by the top encapsulation layer **103, 103'** lining recesses **111** via removable layer **112.**

In sub-step **314 c,** top encapsulation layer **103, 103'** is removed, thereby exposing residual removable layer **112** forming a protective barrier within lined recesses. The protective barrier allows maintaining the integrity of top encapsulation layers **103, 103'** for subsequent processing, in particular when removing of substrate **110.** Advantageously, that approach allows the formation of a double-layer structure **105, 105'** to be formed initially by overlapping top encapsulation layers **103, 103',** and subsequently by applying bottom encapsulation layers **104, 104'** which preferably extend/s over and fully cover/s the entire side walls of the resulting electronic components **101, 101'.** In this way, an improved and highly efficient hermetic encapsulation is enabled.

In sub-step **314d,** residual removable layer **112** is removed, thereby exposing lined recesses **111** for coating with bottom encapsulation layer **104, 104'.** Preferably, removable layer **112** may be removed by exposing layer **112** to conditions or chemicals that are capable of dissolving or otherwise removing removable layer **112.** Generally, removing may involve any suitable physical or chemical means, such as etching, stripping, or other treatment with suitable chemicals or solvents capable of removing removable layer **112.** In case of a photoresist removable layer **112,** removing may preferably comprise: applying light and a photoresist developer, applying a photoresist developer alone or photoresist stripping in wet solutions or a plasma.

Selection of suitable techniques for removing the individual layers during processing is well known. The choice of the appropriate techniques will usually depend on the layer's material to be removed. It will be understood that appropriate removing techniques are typically selected based on the nature of the material to be removed, i.e. each layer is removed by a technique which preferentially or exclusively removes the target layer only rather that other, non-target layers or materials of assembly **100.**

In step **315,** at least one bottom encapsulation layer **104, 104a** is applied to assembly **100,** preferably in upside-down orientation for processing from above, to coat - at the bottom side - electronic components **101, 101'** and recesses **111** lined with top encapsulation layer 103, 103a. Suitable materials for bottom encapsulation layer **104, 104a** are described above. It will be understood that bottom encapsulation layers **104, 104a** may preferably be composed of a conformal material or deposited by techniques that are at least partially directional, which is capable of conforming to the contours of electronic components **101, 101'** and recesses **111** and thereby covers components **101, 101'** and the inner walls of recesses **111** previously lined with top encapsulation layer **103, 103a.** Due to the preferred choice of conformal materials and deposition techniques for bottom encapsulation layer **104, 104a,** a highly satisfying step-like coverage is typically established. As indicated, step **315** may allow applying a monolayer or multilayers of the same or different materials. Step **315** may also comprise - in addition to applying bottom encapsulation layer **104** - a further sub-step of applying at least one additional bottom encapsulation layer **104a** (not shown in step **315**) to assembly **100,** typically after applying bottom encapsulation layer **104.** At least one bottom encapsulation layer **104, 104a** is preferably selected from a corrosion-resistant material allowing to hermetically seal components **101, 101'** against the environment. The outermost bottom encapsulation layer **104, 104a** being the interface to the environment is preferably composed of a biocompatible material in order to reduce or avoid irritation of or affecting the surrounding body cells, tissues or fluids. As indicated, step **315** may comprise adding monolayers or multilayers of the same or different materials.

The inventive method enables top and bottom encapsulation layer/s **103, 103a, 104, 104a** to be preferably applied so as to at least partially overlap and form a double layer structure **105, 105', 105a.** Said double layer structure **105, 105a** preferably at least partially, more preferably fully, covers the electronic components' side walls **106, 106';** in particular, by using removable layer **112** as a protective barrier for top encapsulation layer/s **103, 103a** lining recesses **111** (see **step 314**), and by preferably altering the orientation of the assembly **100** ("flipping") upside-down (allowing upside-down processing of assembly **100**) before applying bottom encapsulation layer **104, 104a.**

One or more of top and/or bottom encapsulation layers **103, 103a, 104, 104**a (or at least a portion thereof) may be patterned using for instance photolithography and etching or lift-off during the process, to introduce e.g. continuous tracks (e.g. at least at the sidewall area) allowing electrical interconnects between the top and bottom side of electronic components **101, 101'.**

By optional step **316,** assembly **100** is secured or fastened to supporting layer **114,** while assembly **100** as provided according to step **315** is released from temporary carrier **113,** preferably subsequently. Securing or bonding may be accomplished by any suitable bonding means, e.g. via a suitable adhesive. The adhesive may preferably be characterized by an adjustable adhesive connection or bonding, which may be modified and, in particular, reduced by applying heat and/or UV light and/or laser light. Supporting layer **114** may preferably be composed of a flexible material, e.g. a thin film, which enables transport or storage of the electronic components 101, 101'. Exemplary materials include flexible polymers, such as so-called semiconductor "dicing tapes". Advantageously, the use of a flexible film allows removing the electronic components **101, 101',** such as dies, by a "die picking" process, whereby the die is pushed from the back using one or multiple pins, and by using a vacuum "pick-up tool" to lift or take off the die from the front. That step is typically carried out by automatic "die picking" machines.

That release from supporting layer 114 is shown in step **317.** The resulting device 1 is encapsulated by top and bottom encapsulating layers **103** and 1**04** resulting from step **315.** Or it is released upon step **316** from its supporting layer **114.** The assembly **100** according to step 317 represents an embodiment of the invention being tightly encapsulated and having the desired hermeticity for its in vivo implantation. It is characterized by a double layer structure 105, 105' formed by encapsulation layers **103** and 1**04** over the entire side wall as a result of the inventive method as shown by the embodiment of **FIG. 2****.**

The inventive method may optionally further comprise step **317** for providing a top coating **107.** Top coating **107** may either be applied prior to applying top encapsulation layer/s **103, 103a** in step **311.** Or, alternatively, top coating **107** may be applied only after applying the top encapsulation layer **103, 103a** in step **311,** e.g. either prior to step **312** or after step **316.** Should the coating step **400** be carried out prior to step **311,** it is carried out before the recesses **111** are introduced into assembly **100** as described above (i.e. onto the assembly **100** of step **309** prior to step **310** of **FIG. 2**) or after the recess has been introduced (applied onto the assembly **100** of step **310** prior to **step 311**). Depending on when the top coating **107** is applied to assembly **100** in the course of the inventive method, the top encapsulation layer/s **103, 103a** may at least partially overlap the coating layer **107** or, alternatively, coating layer **107** may at least partially overlap with encapsulation layers **103, 103a.** When applying top coating **107** by step **400** only after step **316,** it preferably forms a further layer on top of the layers **103, 103a** forming the outermost upper layer of the resulting device **1** being in direct contact with the environment.

Top coating **107** is - according to one embodiment - preferably applied to the electronic components **101, 101'** (or assembly's **100** top side) prior to the application of encapsulation layer(s) **103, 103a.** The application may involve any suitable means, preferably deposition, including chemical vapor deposition (CVD), including plasma-enhanced CVD (PECVD), or physical vapor deposition (PVD), or atomic layer deposition (ALD) or underlying layer oxidation. Subsequently, top coating **107** is preferably partially removed, followed by application of at least one top encapsulation layer/s **103, 103a** such that top coating **107** and encapsulation layer(s) **103, 103a** preferably at least partially overlap with each other. It may be preferred to allow the at least one top encapsulation layer/s **103, 103a** to overlap the coating layer **107** at least at the edge region of the coating layer **107.** Partial removal of top coating **107** is preferably envisaged for exposing the assembly's edges. Removal involves any suitable means, e.g. any chemical or physical process, preferably wet or dry etching and/or lift-off. Partial overlap of top coating **107** and top encapsulation layer/s **103, 103a** due to the resulting packaging hermetically encloses electronic components **101, 101'** without interfering with the intended function of top coating **107.**

Alternatively, top coating **107** may be applied only after applying top encapsulation layer/s **103, 103a** in step **311.** To this end, at least one top encapsulation layer/s **103, 103a** is applied to the electronic components' **101, 101'** from above at their top side. Subsequently, top encapsulation layer/s **103, 103a** is partially removed from electronic component **101, 101'** for top coating **107** to be applied, e.g. at the site of removed portion of the top encapsulation layer **103, 103a.** E.g., top encapsulation layer/s **103, 103a** may be removed from the central portion of the surface of electronic components **101, 101',** while the peripheral portion covering the edges of the electronic components **101, 101'** remains covered by the at least one top encapsulation layer/s **103, 103a.** Any suitable means may be applied, e.g. any chemical or physical process, preferably wet or dry etching and/or lift-off. Thereafter, top coating **107** is applied to electronic components **101, 101',** such that top coating **107** and encapsulation layer/s **103, 103a** preferably at least partially overlap with each other. As above, top coating **107** may be preferably applied by deposition, including chemical vapor deposition (CVD), including PECVD, or physical vapor deposition (PVD), or atomic layer deposition (ALD) or underlying layer oxidation.

The resulting embodiments exhibiting top coating **107** depend on the specific underlying method applied and the stage of the carrying out step **400** in the course of the inventive method. Such alternatives of applying top coating **107** are illustrated in **FIG. 4 A-C.**

All of the embodiments shown in **FIG. 4 A-C.**represent alternative preferred embodiments of step **400** at distinct stages of the inventive method. By step **400** of **Figure 4** **A,** the components **101, 101'** being separated by recess **111** (see step **310** of the method according to **FIG. 2**) are coated (coating layer **107**) at their upper surface and along the inner vertical surface walls of the recess **111** (or rather side walls **106, 106'** of the electronic components **101, 101'** forming recess 111). The coating layer **107** is patterned by etching or liftoff. When using the etching process, the material 107 is deposited first; then, the photoresist is applied and patterned and the material 107 is etched from the regions not covered by photoresist. Finally, the photoresist is removed.

When lift-off is used, the patterning of the photoresist is done before deposition of the material 107; after depositing material 107, the resist and the material 107 over the photoresist is removed to leave layer 107 where the photoresist had previously been removed before depositing material 107.

The embodiment according to step **400** of **Figure 4** **B** is, however, distinct from **Figure 4** **A** in that the coating by coating layer **107** is applied prior to introducing recess **111** (separating electronic components **101, 101'** from each other). The patterning or lift-off process is applied such that recess **111** is only introduced after the patterning or lift-off step **400** according to **FIG. 4** **B.** Both embodiments of **FIG. 4A** and **4** **B** thereafter continue by step **311** (of **Figure 2**) applying the top encapsulating layer **103** to the exposed upper surface and thereby also lining recess **111.** Step **400** according to **FIG. 4** **C** is distinct from step **400** of the embodiments of **Figures 4** **A** and **4 B** by an altered order of steps. For the embodiment of **Figure 4** **C** steps **310** and **311** (of **FIG. 2**) are performed first and only after step **311** (applying the top encapsulation layer **103**) the electronic components **101, 101'** are coated by coating layer **107.** Thus, the embodiment of step **400** according to **Figure 4** **C** allows the coating layer **107** to (partially) overlap the top encapsulating layer **103,** in contrast to the embodiments of **Figures 4** **A** and **4 B.**

It is common to all of these embodiments of step **400** that they may involve etching or lift-off.

In the etching process of step **400,** top coating **107** is applied to the surface of components **101, 101'.** Subsequently, a photoresist layer is applied using suitable methods, e.g. spin coating, which is optionally baked by applying heat. Thereafter, a mask is applied which defines the desired patterning of the resulting top coating **107,** and allows exposing the "masked" photoresist to light. Subsequently, a suitable developer is added which removes the photoresist. In case of positive photoresists, the light-exposed parts are removed. In case of negative photoresists, the non-light exposed parts of the resist are removed. After the "developing step" optional curing or hardening of the resist may be performed by applying heat. Wet or dry etching may follow to selectively remove the parts of top coating **107** which are not covered by the photoresist, while the photoresist-covered parts of top coating **107** are retained. Finally, the remaining resist is removed by stripping in a suitable stripping solution or plasma to yield a top coating **107** which is patterned as defined by the mask.

In the alternative lift-off process for step **400,** photoresist is applied onto the top surface of component **101, 101',** or onto any other layer which is supposed to support top coating 107, e.g. by spin coating. Optionally, it is baked by applying heat. Subsequently, a mask which defines the desired patterning of the resulting top coating **107** is applied to the photoresist, and the "masked" photoresist is exposed to light. Thereafter, a suitable developer is applied which removes the photoresist. In case of positive photoresists, the light-exposed parts are removed. In case of negative photoresists, the non-light exposed parts of the resist are removed. The "developing step" is followed by optionally curing or hardening the resist by applying heat. Top encapsulation layer **103** (or any other layer which is supposed to partially cover top coating **107**) is deposited on the photoresist. Finally, a suitable stripping solution is applied, optionally together with the application of ultra-sound, to lift-off the patterned photoresist together with the covering layer, and to retain the covering layer only where the underlying photoresist was previously removed.

Each of the resulting assemblies **100** shown in **Figures 4** **A** to **4 C** may be further processed according to the inventive method, e.g. by continuing the production process by its step **312** (see **Figure 2**).

Furthermore, the inventive method may include an optional step **500** of providing e.g. electronic traces 108, photodiodes and/or electrodes **109** within the hermetic packaging, e.g. at the top or at the bottom of electronic component **101, 101'.** That optional step **500** is typically carried when the upper surface of electronic component **101** is exposed and accessible to further modification, e.g. at step **309** or **310.** Alternatively, it may be carried out after the outer layers have been applied, e.g. at step **316** or thereafter. That alternative approach is enabled by removing previously applied layer/s or coatings, at least regionwise. Thus, step **500** modifies electronic component **101.** Thereby, electronic traces **108** and/or electrodes **109** may be applied to the surface of electronic components **101, 101',** typically underneath top coating **107** and top encapsulation layer/s **103, 103a,** by any suitable deposition technique, including chemical vapor deposition (CVD), physical vapor deposition, such as e-beam evaporation or (reactive) sputtering, electrochemical deposition and electrodeposition or patterning, including lift-off or etching, as described in more detail below.

Modification of the electronic component **101** by vertically or upward protruding structures (e.g. electrodes, see e.g. **FIG. 1** **D**) may have an impact on the nature of the top encapsulating layer/s **103, 103a** and/or coating layer/s **107.** They may e.g. exhibit holes interrupting one or more of these layer/s. Preferably, electrodes **109** may e.g. be provided within or extending beyond top coating **107** and/or, additionally or alternatively, within or beyond top encapsulation layer/s **103/103a.**

Electrodes **109** may also be introduced only after the provision of the hermetic packaging has been established by locally removing top coating **107** and/or top encapsulation layer/s **103, 103a** so that the surface of components **101, 101'** is typically completely exposed at the electrode deposition sites. The electrodes **109** on the surface of components **101, 101'** may be added, e.g. by depositing a metal or other electrode material layer. First, top coating **107** and/or top encapsulation layer/s **103/103a** may be partially removed to generate holes or feedthroughs for forming electrodes **109.** Removing may be accomplished by any suitable technique, e.g. by etching. Subsequently, electrode material is applied to top coating **107** and/ or top encapsulation layer/s **103/103a.** The excess electrode material is, e.g. subsequently, removed, for instance by etching or lift-off, such that the electrode material is preferably exclusively retained at the sites of the holes or feedthroughs of top coating **107** and/or top encapsulation layer/s **103/103a,** and, optionally, partially overlaps with top coating **107** and/or top encapsulation layer/s **103/103a.** Such an embodiment may be envisaged, whenever electrodes **109** shall be larger in diameter than the hole or feedthrough size.

Alternatively, electrodes **109** may be formed by electronic traces **108** underneath top coating **107** and/or top encapsulation layer/s **103/103a.** To this end, top coating **107** and/or top encapsulation layer/s **103/103a** is/are partially removed, e.g. by etching or lift-off, to form holes or feedthroughs exposing portions of the underlying electronic traces **108.**

The foregoing description details certain embodiments of the invention. It will be appreciated, however, that no matter how detailed the foregoing appears in text, the invention may be practiced in many ways. It should be noted that the use of particular terminology when describing certain features or aspects of the invention should not be taken to imply that the terminology is being re-defined herein to be restricted to including any specific characteristics of the features or aspects of the invention with which that terminology is associated.

While the above detailed description has shown, described, and pointed out novel features of the invention as applied to various embodiments, it will be understood that various omissions, substitutions, and changes in the form and details of the device or process illustrated may be made by those skilled in the technology without departing from the scope of the invention. The scope of the invention is indicated by the appended claims rather than by the foregoing description.

## Claims

1. An implantable device comprising an electronic component (101) encapsulated by a hermetic packaging (10), said packaging comprising a top encapsulation layer (103) and a bottom encapsulation layer (104), wherein top and bottom encapsulation layer (103, 104) at least partially overlap so as to form a double layer (105, 105'),
**characterized in that**
the double layer (105, 105') covers the side walls (106, 106') of the electronic component (101) only, but neither the top side nor the bottom side of electronic component (101).

2. Implantable device according to claim 1, wherein top and/or the bottom encapsulation layer(s) (103, 104) is/are biocompatible,
and/or
wherein top and/or bottom encapsulation layer(s) (103, 104) is/are corrosion-resistant.

3. Implantable device according to claim 2, wherein top and/or bottom encapsulation layer(s) (103, 104) comprise(s) or consist(s) of metal; ceramic including oxides, nitrides, and carbides, preferably metal oxides, metal nitrides and metal carbides; diamond-like carbon; diamond; glass; polymers; combinations thereof, or combinations or multilayers thereof,
wherein said metal is preferably selected from Ti, Pt, stainless steel, titanium-nickel, palladium, niobium, tantalum, combinations or alloys thereof, and multilayers thereof; wherein said ceramic is selected from the group consisting of silicon oxide, silicon nitride, silicon carbide, silicon oxicarbide, titanium carbide, titanium oxide, aluminum oxide, aluminum nitride, zirconium oxide, combinations thereof, and multilayers thereof; and/or said polymer is selected from the group consisting of fluorocarbons, polyurethane, polyether ether ketone (PEEK), PDMS, parylene, polyimide, polycarbonate, polycarbonate urethane, silicone, silicone-polyester-urethane, durimide (photo-definable polyimide), cyclic olefin polymer (COP), cyclic olefin copolymer (COC), polymethyl methacrylate (PMMA), polyphenylene, polysulfone, polyphenylsulfone, combinations thereof and multilayers thereof.

4. Implantable device according to any one of the preceding claims, wherein the packaging further comprises at least one top coating (107), preferably wherein top coating (107) and the top encapsulation layer (103) at least partially or fully overlap with each other,
wherein top coating (107) is preferably biocompatible or wherein top coating (107) is preferably transparent,
or
wherein top coating (107) is preferably corrosion resistant.

5. Implantable device according to claim 4, wherein top coating (107) comprises or consists of a material selected from the group consisting of ceramic; glass including SiC, SiOC; SiO₂; diamond or diamond like carbon; aluminum oxides; titanium oxides; combinations thereof; and multilayers thereof,
and/or
wherein said device, preferably said top coating (107) further comprises electronic traces (108) electronically connected to the electronic component (101), said electronic traces (108) preferably forming electrodes within or protruding from top coating (107),
wherein said electronic traces preferably form electrodes, which are preferably bio-compatible and corrosion-resistant,
and/or
wherein said electronic traces preferably comprise or consist of a material selected from the group consisting of platinum, black/porous platinum, iridium, iridium/platinum, iridium oxide, PEDOT:PSS, titanium nitride, doped diamond or doped diamond like carbon and graphene, and combinations thereof.

6. Implantable device according to any one of the preceding claims, wherein said electronic component (101) encapsulated by said hermetic packaging (10) comprises a layer (102),
wherein layer (102) preferably comprises or preferably consists of ceramics or glass, optionally selected from silicon oxide, optionally obtained by oxidation of a silicon substrate.

7. Implantable device according to any one of the preceding claims, (i) further comprising at least one additional top encapsulation layer (103a) on top of top encapsulation layer (103) and/or at least one additional bottom encapsulation layer (104a) encapsulating bottom encapsulation layer (104),
(ii) wherein further encapsulation layers (103a, 104a) preferably overlap so as to form at least one additional double layer (105a, 105b, 105c) optionally covering at least partially, more preferably fully, side walls (106, 106') of electronic component (101),
(iii) wherein the at least one additional double layer (105a, 105b, 105c) more preferably covers the side walls (106, 106') only, but neither the top side nor the bottom side of the electronic component (101).

8. Implantable device according to any one of claims 7(i) or 7(iii), wherein top and/or bottom encapsulation layer(s) (103, 103', 104, 104') is/are corrosion resistant and optionally bio-compatible,
and/or
wherein the at least one additional top and/or bottom encapsulation layer(s) (103a, 104a) is/are biocompatible and optionally corrosion-resistant.

9. Implantable device according to any one of the preceding claims, wherein top and/or bottom encapsulation layer(s) (103, 104) and/or optionally further top and/or bottom encapsulation layer(s) (103a, 104a) comprise(s) or consist(s) of the same or a different material.

10. Implantable device according to any one of the preceding claims, wherein the implantable device comprises photodiodes and/or electrodes (109) being exposed to the environment, preferably embedded by a top coating layer and/or a top encapsulation layer such that their outer top surface is exposed to the environment.

11. Implantable device according to any one of the preceding claims, wherein the implantable device is configured for being implantable in the eye, preferably as a retinal implant being configured for being implantable epi- or subretinally.

12. An implantable system comprising at least one packaged device according to any of claims 1 to 11.

13. A method for packaging an implantable device, the method comprising
(a) providing at least one electronic component (101) or an assembly (100) on a substrate (110);
(b) applying at least one top encapsulation layer (103, 103') to electronic component (101, 101') or an assembly (100); and
(c) applying at least one bottom encapsulation layer (104, 104') to electronic component (101, 101') or an assembly (100);
wherein the top and bottom encapsulation layer (103, 103', 104, 104') at least partially overlap so as to form a double layer (105, 105', 105a, 105b, 105c), **characterized in that**
the double layer (105, 105') is provided such that the double layer (105, 105') covers the side walls (106, 106') only, but neither the top side nor the bottom side of the electronic component (101).

14. The method for packaging an implantable device according to claim 13, said method comprising the steps of:
(i) providing an assembly (100) of at least one, preferably a plurality of electronic components (101, 101') spaced apart from each other on a substrate (110), wherein adjacent electronic components (101, 101') and substrate (110) define recess (111) in between electronic components (101, 101');
(ii) applying at least one top encapsulation layer (103, 103') to assembly (100), thereby coating electronic components (101, 101') and lining recess (111);
(iii) applying a removable layer (112) to assembly (100);
(iv) partially removing removable layer (112), thereby leaving a residual amount of removable layer (112) within lined recess (111);
(v) preferably upending or flipping assembly (100) upside down;
(vi) removing (a) substrate (110), (b) top encapsulation layer (103) and (c) residual amount of removable layer (112) from the assembly's bottom side; and
(vii) applying at least one bottom encapsulation layer (104, 104') to assembly (100), thereby preferably coating electronic components (101, 101') and recess (111), wherein the top and bottom encapsulation layer (103, 103', 104, 104') are applied so as to at least partially overlap and forming a double layer (105, 105').

15. The method according to any one of claims 13 to 14, further comprising a step of providing a top coating (107) by
(a) applying a top coating (107), preferably as defined in any one of the preceding claims, to the top side of electronic component (101, 101'), (b) partially removing top coating (107), and (c) applying at least one top encapsulation layer (103, 103'), preferably as defined in any one of the preceding claims, to the electronic component (101, 101'), such that top coating (107) and encapsulation layer(s) (103, 103') preferably at least partially overlap with each other;
or
(a') applying at least one top encapsulation layer (103, 103'), preferably as defined in any one of the preceding claims, to electronic component (101, 101'), (b') partially removing top encapsulation layer(s) (103, 103') from electronic component (101, 101'), and (c') applying a top coating (107), preferably as defined in any one of the preceding claims, to electronic component (101, 101'), such that top coating (107) and encapsulation layer(s) (103, 103') preferably at least partially overlap with each other,
wherein step (b) preferably includes partially removing top coating (107) or (b') preferably includes partially removing top encapsulation layer(s) (103, 103') by any chemical or physical process, more preferably wet or dry etching and/or lift-off,
and/or
wherein top coating (107) is preferably applied by deposition, including chemical vapor deposition (CVD), including PECVD, or physical vapor deposition (PVD), or atomic layer deposition (ALD) or underlying layer oxidation.

16. The method according to any one of claims 13 to 15, further comprising a step of providing electronic traces (108) and/or electrodes (109), preferably within top coating (107), by deposition, including physical vapor deposition or electrodeposition, and/or patterning, including lift-off or etching.

17. The method according to any one of claims 14 to 16, further comprising prior to step (iv) a step of attaching the assembly's top side to a temporary carrier (113),
and/or
wherein the substrate (110), top encapsulation layer (103) and/or removable layer (112) is/are removed by means each independently selected from physical and chemical means, including grinding, etching and/or stripping.

18. The method according to any one of claims 13 to 17, wherein top and/or bottom encapsulation layer(s) (103, 104) is/are biocompatible,
and/or
wherein top and/or bottom encapsulation layer(s) (103, 103a, 104, 104a) is/are corrosion-resistant,
and/or
wherein top and/or the bottom encapsulation layer(s)(103, 103a, 104, 104a) comprise(s) or consist(s) of a material optionally selected from a metal, including titanium, platinum, stainless steel, titanium-nickel, palladium, niobium, tantalum, alloys and multilayers thereof; a ceramic, including metal oxides, metal nitrides, and metal carbides such as silicon oxide, silicon nitride, silicon carbide, silicon oxicarbide, titanium carbide, aluminum oxide, aluminum nitride, zirconium oxide and multilayers thereof; diamond-like carbon; diamond; glass; low permeability and/or dense polymers, including fluorocarbons, polyurethane, PEEK, silicone, PDMS, parylene, polyimide; or multilayers thereof.

19. The method according to any one of claims 15 to 18, wherein removable layer (112) comprises or consists of a material selected from a polymeric material, preferably a resin, more preferably a photosensitive resin, and a dissolvable polymeric material.

20. The method according to any one of claims 15 to 19, wherein top coating (107) is biocompatible,
and/or
wherein top coating (107) is corrosion-resistant,
and/or
wherein top coating (107) is transparent,
and/or
wherein top coating (107) comprises or consists of a material selected from ceramics; glass, including PECVD SiC, SiOC, SiO2; diamond or diamond like carbon; aluminum oxides; titanium oxides; or multilayers thereof.

21. The method according to any one of claims 13 to 20, wherein the method provides a packaged device according to any of claim 1 to 11.

## Patentansprüche

1. Eine implantierbare Vorrichtung, die ein elektronisches Bauteil (101) umfasst, das von einer hermetischen Verpackung (10) eingekapselt ist, wobei die Verpackung eine obere Einkapselungsschicht (103) und eine untere Einkapselungsschicht (104) umfasst, wobei die obere und die untere Einkapselungsschicht (103, 104) zumindest teilweise überlappen, um eine Doppelschicht (105, 105') zu bilden,
**dadurch gekennzeichnet, dass**
die Doppelschicht (105, 105') nur die Seitenwände (106, 106') des elektronischen Bauteils (101) bedeckt, aber weder die Oberseite noch die Unterseite des elektronischen Bauteils (101).

2. Implantierbare Vorrichtung nach Anspruch 1, wobei die obere(n) und/oder die untere(n) Einkapselungsschicht(en) (103, 104) biokompatibel ist/sind,
und/ oder
wobei die obere und/oder untere Einkapselungsschicht(en) (103, 104) korrosionsbeständig ist/sind.

3. Implantierbare Vorrichtung nach Anspruch 2, wobei die obere(n) und/oder untere(n) Einkapselungsschicht(en) (103, 104) Metall; Keramik, einschließlich Oxiden, Nitriden und Carbiden, vorzugsweise Metalloxiden, Metallnitriden und Metallcarbiden; diamantartigem Kohlenstoff; Diamant; Glas; Polymeren; Kombinationen davon oder Kombinationen oder Mehrfachschichten davon umfassen oder aus Metall; Keramik, einschließlich Oxiden, Nitriden und Carbiden, vorzugsweise Metalloxiden, Metallnitriden und Metallcarbiden; diamantartigem Kohlenstoff; Diamant; Glas; Polymeren; Kombinationen davon oder Kombinationen oder Mehrfachschichten davon besteht oder bestehen,
wobei das Metall vorzugsweise ausgewählt ist aus Ti, Pt, rostfreiem Stahl, Titan-Nickel, Palladium, Niob, Tantal, Kombinationen oder Legierungen davon und Mehrfachschichten davon; wobei die Keramik ausgewählt ist aus der Gruppe bestehend aus Siliziumoxid, Siliziumnitrid, Siliziumcarbid, Siliziumoxicarbid, Titancarbid, Titanoxid, Aluminiumoxid, Aluminiumnitrid, Zirkoniumoxid, Kombinationen davon und Mehrfachschichten davon; und/oder das Polymer ausgewählt ist aus der Gruppe bestehend aus Fluorkohlenstoffen, Polyurethan, Polyetheretherketon (PEEK), PDMS, Parylen, Polyimid, Polycarbonat, Polycarbonat-Urethan, Silikon, Silikon-Polyester-Urethan, Durimid (photodefinierbares Polyimid), zyklisches Olefinpolymer (COP), zyklisches Olefin-Copolymer (COC), Polymethylmethacrylat (PMMA), Polyphenylen, Polysulfon, Polyphenylsulfon, Kombinationen davon und Mehrfachschichten davon.

4. Implantierbare Vorrichtung nach einem der vorhergehenden Ansprüche, wobei die Verpackung ferner mindestens eine obere Beschichtung (107) umfasst, wobei vorzugsweise die obere Beschichtung (107) und die obere Einkapselungsschicht (103) miteinander zumindest teilweise oder vollständig überlappen,
wobei die obere Beschichtung (107) vorzugsweise biokompatibel ist oder wobei die obere Beschichtung (107) vorzugsweise transparent ist,
oder
wobei die obere Beschichtung (107) vorzugsweise korrosionsbeständig ist.

5. Implantierbare Vorrichtung nach Anspruch 4, wobei die obere Beschichtung (107) ein Material umfasst oder aus einem Material besteht, das aus der Gruppe ausgewählt ist, die aus Keramik; Glas, einschließlich SiC, SiOC, SiO₂; Diamant oder diamantähnlichem Kohlenstoff; Aluminiumoxiden; Titanoxiden; Kombinationen davon; und Mehrfachschichten davon besteht,
und/ oder
wobei die Vorrichtung, vorzugsweise die obere Beschichtung (107), ferner elektronische Leiterbahnen (108) umfasst, die elektronisch mit dem elektronischen Bauteil (101) verbunden sind, wobei die elektronischen Leiterbahnen (108) vorzugsweise Elektroden innerhalb der oberen Beschichtung (107) bilden oder aus dieser herausragen,
wobei die elektronischen Leiterbahnen vorzugsweise Elektroden bilden, die vorzugsweise biokompatibel und korrosionsbeständig sind,
und/oder
wobei die elektronischen Leiterbahnen vorzugsweise ein Material umfassen oder aus einem Material bestehen, das aus der Gruppe ausgewählt ist, die aus Platin, schwarzem/porösem Platin, Iridium, Iridium/Platin, Iridiumoxid, PEDOT:PSS, Titannitrid, dotiertem Diamant oder dotiertem diamantähnlichem Kohlenstoff und Graphen sowie Kombinationen davon besteht.

6. Implantierbare Vorrichtung nach einem der vorhergehenden Ansprüche, wobei das von der hermetischen Verpackung (10) eingekapselte elektronische Bauteil (101) eine Schicht (102) umfasst,
wobei die Schicht (102) vorzugsweise Keramik oder Glas umfasst oder vorzugsweise daraus besteht, optional ausgewählt aus Siliziumoxid, optional erhalten durch Oxidation eines Siliziumsubstrats.

7. Implantierbare Vorrichtung nach einem der vorhergehenden Ansprüche, (i) ferner umfassend mindestens eine zusätzliche obere Einkapselungsschicht (103a) auf der oberen Einkapselungsschicht (103) und/oder mindestens eine zusätzliche untere Einkapselungsschicht (104a), die die untere Einkapselungsschicht (104) einkapselt,
(ii) wobei weitere Einkapselungsschichten (103a, 104a) vorzugsweise so überlappen, dass sie mindestens eine zusätzliche Doppelschicht (105a, 105b, 105c) bilden, die optional zumindest teilweise, vorzugsweise vollständig, Seitenwände (106, 106') des elektronischen Bauteils (101) bedecken,
(iii) wobei die mindestens eine zusätzliche Doppelschicht (105a, 105b, 105c) stärker vorzugsweise nur die Seitenwände (106, 106'), aber weder die Oberseite noch die Unterseite des elektronischen Bauteils (101) bedeckt.

8. Implantierbare Vorrichtung nach einem der Ansprüche 7 (i) oder 7 (iii), wobei die obere(n) und/oder untere(n) Einkapselungsschicht/en (103, 103', 104, 104') korrosionsbeständig und optional biokompatibel ist/sind,
und/oder
wobei die mindestens eine zusätzliche(n) obere(n) und/oder untere(n) Einkapselungsschicht(en) (103a, 104a) biokompatibel und optional korrosionsbeständig ist/sind.

9. Implantierbare Vorrichtung nach einem der vorhergehenden Ansprüche, wobei obere und/oder untere Einkapselungsschicht(en) (103, 104) und/oder optional weitere obere und/oder untere Einkapselungsschicht(en) (103a, 104a) dasselbe oder ein anderes Material umfassen oder aus demselben oder einem anderen Material bestehen.

10. Implantierbare Vorrichtung nach einem der vorhergehenden Ansprüche, wobei die implantierbare Vorrichtung Photodioden und/oder Elektroden (109) umfasst, die der Umgebung ausgesetzt sind, vorzugsweise eingebettet von einer oberen Beschichtungsschicht und/oder einer oberen Einkapselungsschicht, so dass ihre äußere obere Oberfläche der Umgebung ausgesetzt ist.

11. Implantierbare Vorrichtung nach einem der vorhergehenden Ansprüche, wobei die implantierbare Vorrichtung so konfiguriert ist, dass sie in das Auge implantiert werden kann, vorzugsweise als Netzhautimplantat, das so konfiguriert ist, dass es epi- oder subretinal implantiert werden kann.

12. Ein implantierbares System, umfassend mindestens eine verpackte Vorrichtung nach einem der Ansprüche 1 bis 11.

13. Ein Verfahren zum Verpacken einer implantierbaren Vorrichtung, wobei das Verfahren umfasst
(a) Bereitstellen mindestens eines elektronischen Bauteils (101) oder einer Baugruppe (100) auf einem Substrat (110);
(b) Aufbringen mindestens einer oberen Einkapselungsschicht (103, 103') auf ein elektronisches Bauteil (101, 101') oder eine Baugruppe (100); und
(c) Aufbringen mindestens einer unteren Einkapselungsschicht (104, 104') auf ein elektronisches Bauteil (101, 101') oder eine Baugruppe (100);
wobei sich die obere und die untere Einkapselungsschicht (103, 103', 104, 104') zumindest teilweise überlappen, so dass sie eine Doppelschicht (105, 105', 105a, 105b, 105c) bilden,
**dadurch gekennzeichnet, dass**
die Doppelschicht (105, 105') so bereitgestellt, dass die Doppelschicht (105, 105') nur die Seitenwände (106, 106'), aber weder die Oberseite noch die Unterseite des elektronischen Bauteils (101) bedeckt.

14. Verfahren zum Verpacken einer implantierbaren Vorrichtung nach Anspruch 13, wobei das Verfahren die folgenden Schritte umfasst:
(i) Bereitstellen einer Baugruppe (100) aus mindestens einem, vorzugsweise einer Vielzahl von elektronischen Bauteilen (101, 101'), die voneinander beabstandet auf einem Substrat (110) angeordnet sind, wobei benachbarte elektronische Bauteile (101, 101') und das Substrat (110) eine Aussparung (111) zwischen den elektronischen Bauteilen (101, 101') definieren;
(ii) Aufbringen mindestens einer oberen Einkapselungsschicht (103, 103') auf die Baugruppe (100), wodurch die elektronischen Bauteile (101, 101') beschichtet und die Aussparung (111) ausgekleidet werden;
(iii) Aufbringen einer entfernbaren Schicht (112) auf die Baugruppe (100);
(iv) teilweises Entfernen der entfernbaren Schicht (112), wodurch eine Restmenge der entfernbaren Schicht (112) innerhalb der ausgekleideten Aussparung (111) verbleibt;
(v) vorzugsweise Umdrehen oder Auf-den-Kopf-Stellen der Baugruppe (100);
(vi) Entfernen (a) des Substrats (110), (b) der oberen Einkapselungsschicht (103) und (c) der Restmenge der entfernbaren Schicht (112) von der Unterseite der Baugruppe; und
(vii) Aufbringen mindestens einer unteren Einkapselungsschicht (104, 104') auf die Baugruppe (100), wodurch vorzugsweise elektronische Bauteile (101, 101') und die Aussparung (111) beschichtet werden,
wobei die obere und die untere Einkapselungsschicht (103, 103', 104, 104') so aufgebracht werden, dass sie sich zumindest teilweise überlappen und eine Doppelschicht (105, 105') bilden.

15. Verfahren nach einem der Ansprüche 13 bis 14, das ferner einen Schritt der Bereitstellung einer oberen Beschichtung (107) umfasst, indem
(a) Aufbringen einer oberen Beschichtung (107), vorzugsweise wie in einem der vorangehenden Ansprüche definiert, auf die Oberseite des elektronischen Bauteils (101, 101'), (b) teilweises Entfernen der oberen Beschichtung (107), und (c) Aufbringen mindestens einer oberen Einkapselungsschicht (103, 103'), vorzugsweise wie in einem der vorangehenden Ansprüche definiert, auf das elektronische Bauteil (101, 101'), so dass die obere Beschichtung (107) und die Einkapselungsschicht(en) (103, 103') vorzugsweise zumindest teilweise miteinander überlappen;
oder
(a') Aufbringen mindestens einer oberen Einkapselungsschicht (103, 103'), vorzugsweise wie in einem der vorangehenden Ansprüche definiert, auf das elektronische Bauteil (101, 101'), (b') teilweises Entfernen der oberen Einkapselungsschicht(en) (103, 103') vom elektronischen Bauteil (101, 101'), und (c') Aufbringen einer oberen Beschichtung (107), vorzugsweise wie in einem der vorangehenden Ansprüche definiert, auf das elektronische Bauteil (101, 101'), so dass die obere Beschichtung (107) und die Einkapselungsschicht(en) (103, 103') vorzugsweise zumindest teilweise miteinander überlappen,
wobei Schritt (b) vorzugsweise das teilweise Entfernen der oberen Beschichtung (107) oder (b') vorzugsweise das teilweise Entfernen der oberen Einkapselungsschicht(en) (103, 103') durch ein beliebiges chemisches oder physikalisches Verfahren, vorzugsweise Nass- oder Trockenätzen und/oder Abheben, umfasst,
und/oder
wobei die obere Beschichtung (107) vorzugsweise durch Abscheidung, einschließlich chemischer Gasphasenabscheidung (CVD), einschließlich PECVD, oder physikalischer Gasphasenabscheidung (PVD), oder Atomlagenabscheidung (ALD) oder Oxidation der darunter liegenden Schicht aufgebracht wird.

16. Verfahren nach einem der Ansprüche 13 bis 15, das ferner einen Schritt der Bereitstellung elektronischer Leiterbahnen (108) und/oder Elektroden (109), vorzugsweise innerhalb der oberen Beschichtung (107), durch Abscheidung, einschließlich physikalischer Dampfabscheidung oder Elektroabscheidung, und/oder Strukturierung, einschließlich Abheben oder Ätzen, umfasst.

17. Verfahren nach einem der Ansprüche 14 bis 16, weiter umfassend vor dem Schritt (iv) einen Schritt des Anordnens der Oberseite der Baugruppe auf einem temporären Träger (113),
und/ oder
wobei das Substrat (110), die obere Einkapselungsschicht (103) und/oder die entfernbare Schicht (112) durch Mittel entfernt wird/werden, die jeweils unabhängig voneinander aus physikalischen und chemischen Mitteln ausgewählt werden, einschließlich Schleifen, Ätzen und/oder Ablösen.

18. Verfahren nach einem der Ansprüche 13 bis 17, wobei die obere(n) und/oder untere(n) Einkapselungsschicht(en) (103, 104) biokompatibel ist/ sind,
und/oder
wobei die obere(n) und/oder untere(n) Einkapselungsschicht(en) (103, 103a, 104, 104a) korrosionsbeständig ist/ sind,
und/ oder
wobei die obere(n) und/oder die untere(n) Einkapselungsschicht(en) (103, 103a, 104, 104a) ein Material umfasst/en oder aus einem Material besteht/en, das optional aus einem Metall, einschließlich Titan, Platin, rostfreiem Stahl, Titan-Nickel, Palladium, Niob, Tantal, Legierungen und Mehrfachschichten davon; Keramik, einschließlich Metalloxiden, Metallnitriden und Metallcarbiden wie Siliziumoxid, Siliziumnitrid, Siliziumcarbid, Siliziumoxicarbid, Titancarbid, Aluminiumoxid, Aluminiumnitrid, Zirkoniumoxid und Mehrfachschichten davon; diamantartigem Kohlenstoff; Diamant; Glas; gering durchlässigen und/oder dichten Polymeren, einschließlich Fluorkohlenstoffen, Polyurethan, PEEK, Silikon, PDMS, Parylen, Polyimid; oder Mehrfachschichten davon ausgewählt ist.

19. Verfahren nach einem der Ansprüche 15 bis 18, wobei die entfernbare Schicht (112) ein Material umfasst oder aus einem Material besteht, das aus einem polymeren Material, vorzugsweise einem Harz, besonders bevorzugt einem lichtempfindlichen Harz, und einem auflösbaren polymeren Material ausgewählt ist.

20. Verfahren nach einem der Ansprüche 15 bis 19, wobei die obere Beschichtung (107) biokompatibel ist,
und/ oder
wobei die obere Beschichtung (107) korrosionsbeständig ist,
und/ oder
wobei die obere Beschichtung (107) transparent ist,
und/ oder
wobei die obere Beschichtung (107) ein Material umfasst oder aus einem Material besteht, das ausgewählt ist aus Keramik; Glas, einschließlich PECVD-SiC, SiOC, SiO2; Diamant oder diamantähnlichem Kohlenstoff; Aluminiumoxiden; Titanoxiden; oder Mehrfachschichten davon.

21. Verfahren nach einem der Ansprüche 13 bis 20, wobei das Verfahren eine verpackte Vorrichtung nach einem der Ansprüche 1 bis **11** bereitstellt.

## Revendications

1. Dispositif implantable comprenant un composant électronique (101) encapsulé par un enrobage hermétique (10), ledit enrobage comprenant une couche d'encapsulation supérieure (103) et une couche d'encapsulation inférieure (104), dans lequel les couches d'encapsulation supérieure et inférieure (103, 104) se chevauchent au moins partiellement, de façon à former une double couche (105, 105'),
**caractérisé en ce que**
la double couche (105, 105') couvre les parois latérales (106, 106') du composant électronique (101) uniquement, mais ni le côté supérieur ni le côté inférieur du composant électronique (101).

2. Dispositif implantable selon la revendication 1, dans lequel la/les couche(s) d'encapsulation supérieure et/ou inférieure (103, 104) est/sont biocompatibles,
et/ou
dans lequel la/les couche(s) d'encapsulation supérieure et/ou inférieure (103, 104) est/sont résistante(s) à la corrosion.

3. Dispositif implantable selon la revendication 2, dans lequel la/les couche(s) d'encapsulation supérieure et/ou inférieure (103, 104) comprend/comprennent ou est/sont constituée(s) de métal ; céramique y compris oxydes, nitrures et carbures, de préférence oxydes métalliques, nitrures métalliques et carbures métalliques ; carbone adamantin ; diamant ; verre ; polymères ; combinaisons de ceux-ci, ou combinaisons ou multicouches de ceux-ci,
dans lequel ledit métal est de préférence choisi parmi le Ti, le Pt, l'acier inoxydable, le nickel-titane, le palladium, le niobium, le tantale, les combinaisons ou alliages de ceux-ci, et les multicouches de ceux-ci ; dans lequel la céramique est choisie dans le groupe constitué par l'oxyde de silicium, le nitrure de silicium, le carbure de silicium, l'oxycarbure de silicium, le carbure de titane, l'oxyde de titane, l'oxyde d'aluminium, le nitrure d'aluminium, l'oxyde de zirconium, les combinaisons de ceux-ci, et les multicouches de ceux-ci ; et/ou ledit polymère est choisi dans le groupe constitué par les fluorocarbones, le polyuréthane, le polyétheréthercétone (PEEK), le PDMS, le parylène, le polyimide, le polycarbonate, le polycarbonate uréthane, le silicone, le silicone-polyester-uréthane, le durimide (polyimide photo-définissable), le polymère oléfinique cyclique (POC), le copolymère oléfinique cyclique (COC), le polyméthylméthacrylate (PMMA), le polyphénylène, le polysulfone, le polyphénylsulfone, les combinaisons de ceux-ci et les multicouches de ceux-ci.

4. Dispositif implantable selon l'une quelconque des revendications précédentes, dans lequel l'enrobage comprend en outre au moins un revêtement supérieur (107), de préférence dans lequel le revêtement supérieur (107) et la couche d'encapsulation supérieure (103) se chevauchent au moins partiellement ou complètement,
dans lequel le revêtement supérieur (107) est de préférence biocompatible ou dans lequel le revêtement supérieur (107) est de préférence transparent, ou
dans lequel le revêtement supérieur (107) est de préférence résistant à la corrosion.

5. Dispositif implantable selon la revendication 4, dans lequel le revêtement supérieur (107) comprend ou est constitué d'un matériau choisi dans le groupe constitué par la céramique ; le verre y compris le SiC, SiOC ; le SiO₂ ; le diamant ou le carbone adamantin ; les oxydes d'aluminium ; les oxydes de titane ; les combinaisons de ceux-ci ; et les multicouches de ceux-ci,
et/ou
dans lequel dispositif, de préférence ledit revêtement supérieur (107) comprend des traces électroniques (108) électroniquement connectées au composant électronique (101), lesdites traces électroniques (108) formant de préférence des électrodes rentrantes ou saillantes par rapport au revêtement supérieur (107),
dans lequel lesdites traces électroniques forment de préférence des électrodes, qui sont de préférence biocompatibles et résistantes à la corrosion,
et/ou
dans lequel lesdites traces électroniques comprennent ou sont constituées de préférence d'un matériau choisi dans le groupe constitué par le platine, le noir de platine/platine poreux, l'iridium, l'iridium/platine, l'oxyde d'iridium, le PEDOT:PSS, le nitrure de titane, le diamant dopé ou le carbone adamantin dopé et le graphène, et les combinaisons de ceux-ci.

6. Dispositif implantable selon l'une quelconque des revendications précédentes, dans lequel ledit composant électronique (101) encapsulé par ledit enrobage hermétique (10) comprend une couche (102),
dans lequel la couche (102) comprend de préférence ou est constituée de préférence de céramique ou de verre, facultativement choisi parmi l'oxyde de silicium, facultativement obtenu par oxydation d'un substrat à base de silicium.

7. Dispositif implantable selon l'une quelconque des revendications précédentes,
(i) comprenant en outre au moins une couche d'encapsulation supérieure supplémentaire (103a) au-dessus de la couche d'encapsulation supérieure (103) et/ou au moins une couche d'encapsulation inférieure supplémentaire (104a) encapsulant la couche d'encapsulation inférieure (104),
(ii) dans lequel les couches d'encapsulation supplémentaires (103a, 104a) de préférence se chevauchent, de façon à former au moins une double couche supplémentaire (105a, 105b, 105c) couvrant facultativement au moins partiellement, plus préférablement complètement, les parois latérales (106, 106') du composant électronique (101),
(iii) dans lequel l'au moins une double couche supplémentaire (105a, 105b, 105c) couvre plus préférablement les parois latérales (106, 106') uniquement, mais ni le côté supérieur ni le côté inférieur du composant électronique (101).

8. Dispositif implantable selon l'une quelconque des revendications 7(i) ou 7(iii), dans lequel la/les couche(s) d'encapsulation supérieure et/ou inférieure (103, 103', 104, 104') est/sont résistante(s) à la corrosion et facultativement biocompatible(s),
et/ou
dans lequel l'au moins une/des couche(s) d'encapsulation supérieure et/ou inférieure supplémentaire(s) (103a, 104a) est/sont biocompatible(s) et facultativement résistante(s) à la corrosion.

9. Dispositif implantable selon l'une quelconque des revendications précédentes, dans lequel la/les couche(s) d'encapsulation supérieure et/ou inférieure (103, 104) et/ou facultativement la/les couche(s) d'encapsulation supérieure et/ou inférieure supplémentaire(s) (103a, 104a) comprend/comprennent ou est/sont constituée(s) d'un matériau identique ou différent.

10. Dispositif implantable selon l'une quelconque des revendications précédentes, dans lequel le dispositif implantable comprend des photodiodes et/ou électrodes (109) qui sont exposées à l'environnement, de préférence incorporées dans une couche de revêtement supérieure et/ou une couche d'encapsulation supérieure de sorte que leur surface supérieure extérieure est exposée à l'environnement.

11. Dispositif implantable selon l'une quelconque des revendications précédentes, dans lequel le dispositif implantable est configuré pour être implantable dans l'œil, de préférence sous forme d'implant rétinien configuré pour être implantable par voie épirétinienne ou sous-rétinienne.

12. Système implantable comprenant au moins un dispositif enrobé selon l'une quelconque des revendications 1 à 11.

13. Procédé d'enrobage d'un dispositif implantable, le procédé comprenant
(a) la fourniture d'au moins un composant électronique (101) ou d'un assemblage (100) sur un substrat (110) ;
(b) l'application d'au moins une couche d'encapsulation supérieure (103, 103') sur le composant électronique (101, 101') ou sur un assemblage (100) ; et
(c) l'application d'au moins une couche d'encapsulation inférieure (104, 104') sur le composant électronique (101, 101') ou sur un assemblage (100) ;
dans lequel les couches d'encapsulation supérieure et inférieure (103, 103', 104, 104') se chevauchent au moins partiellement, de façon à former une double couche (105, 105', 105a, 105b, 105c),
**caractérisé en ce que**
la double couche (105, 105') est fournie de sorte que la double couche (105, 105') couvre les parois latérales (106, 106') uniquement, mais ni le côté supérieur ni le côté inférieur du composant électronique (101).

14. Procédé d'enrobage d'un dispositif implantable selon la revendication 13, ledit procédé comprenant les étapes de :
(i) fourniture d'un assemblage (100) d'au moins un, de préférence une pluralité de composants électroniques (101, 101') espacés les uns des autres sur un substrat (110), dans lequel les composants électroniques adjacents (101, 101') et le substrat (110) définissent des renfoncements (111) entre les composants électroniques (101, 101') ;
(ii) application d'au moins une couche d'encapsulation supérieure (103, 103') sur l'assemblage (100), revêtant ainsi les composants électroniques (101, 101') et recouvrant le renfoncement (111) ;
(iii) application d'une couche amovible (112) sur l'assemblage (100) ;
(iv) élimination partielle de la couche amovible (112), laissant ainsi une quantité résiduelle de couche amovible (112) dans le renfoncement recouvert (111) ;
(v) de préférence redressement ou renversement de l'assemblage (100) ;
(vi) élimination (a) du substrat (110), (b) de la couche d'encapsulation supérieure (103) et (c) de la quantité résiduelle de couche amovible (112) du côté inférieur de l'assemblage ; et
(vii) application d'au moins une couche d'encapsulation inférieure (104, 104') sur l'assemblage (100), revêtant ainsi de préférence les composants électroniques (101, 101') et le renfoncement (111), dans lequel les couches d'encapsulation supérieure et ensuite inférieure (103, 103', 104, 104') sont appliquées de façon à se chevaucher au moins partiellement et à former une double couche (105, 105').

15. Procédé selon l'une quelconque des revendications 13 à 14, comprenant en outre une étape de fourniture d'un revêtement supérieure (107) par (a) application d'un revêtement supérieure (107), de préférence tel que défini dans l'une quelconque des revendications précédentes, sur le côté supérieur du composant électronique (101, 101'), (b) élimination partielle du revêtement supérieur (107), et (c) application d'au moins une couche d'encapsulation supérieure (103, 103'), de préférence telle que définie dans l'une quelconque des revendications précédentes, sur le composant électronique (101, 101'), de sorte que le revêtement supérieur (107) et la/les couche(s) d'encapsulation (103, 103') se chevauchent de préférence au moins partiellement ;
ou
(a') application d'au moins une/des couche(s) d'encapsulation supérieure(s) (103, 103'), de préférence telle(s) que définie(s) dans l'une quelconque des revendications précédentes, sur le composant électronique (101, 101'), (b') élimination partielle de la/des couche(s) d'encapsulation supérieure(s) (103, 103') du composant électronique (101, 101'), et (c') application d'un revêtement supérieur (107), de préférence tel que défini dans l'une quelconque des revendications précédentes, sur le composant électronique (101, 101'), de sorte que le revêtement supérieur (107) et la/les couche(s) d'encapsulation (103, 103') se chevauchent de préférence au moins partiellement ;
dans lequel l'étape (b) comprend de préférence l'élimination partielle du revêtement supérieur (107) ou (b') comprend de préférence l'élimination partielle de la/des couche(s) d'encapsulation supérieure(s) (103, 103') par un quelconque procédé chimique ou physique, plus préférablement la gravure humide ou sèche et/ou le décollage,
et/ou
dans lequel le revêtement supérieur (107) est de préférence appliqué par dépôt, y compris le dépôt chimique en phase vapeur (CVD), notamment le PECVD, le dépôt physique en phase vapeur (PVD), le dépôt de couches atomiques (ALD) ou l'oxydation de couches sous-jacentes.

16. Procédé selon l'une quelconque des revendications 13 à 15, comprenant en outre une étape de fourniture de traces électroniques (108) et/ou d'électrodes (109), de préférence dans un revêtement supérieur (107), par dépôt, y compris le dépôt physique en phase vapeur ou le dépôt électrolytique ; et/ou la structuration, y compris le décollage ou la gravure.

17. Procédé selon l'une quelconque des revendications 14 à 16, comprenant en outre, avant l'étape (iv), une étape de fixation du côté supérieur de l'assemblage à un support temporaire (113),
et/ou
dans lequel le substrat (110), la couche d'encapsulation supérieure (103) et/ou la couche amovible (112) sont éliminés par un moyen chacun indépendamment choisi parmi un moyen physique et chimique, y compris le meulage, la gravure et/ou le décapage.

18. Procédé selon l'une quelconque des revendications 13 à 17, dans lequel la/les couche(s) d'encapsulation supérieure et/ou inférieure (103, 104) est/sont biocompatible(s),
et/ou
dans lequel la/les couche(s) d'encapsulation supérieure et/ou inférieure (103, 103a, 104, 104a) est/sont résistante(s) à la corrosion,
et/ou
dans lequel la/les couche(s) d'encapsulation supérieure et/ou inférieure (103, 103a, 104, 104a) comprend/comprennent ou est/sont constituée(s) d'un matériau choisi parmi un métal, y compris le titane, le platine, l'acier inoxydable, le nickel-titane, le palladium, le niobium, le tantale, les alliages et multicouches de ceux-ci ; une céramique, y compris les oxydes métalliques, les nitrures métalliques et les carbures métalliques tels que l'oxyde de silicium, le nitrure de silicium, le carbure de silicium, l'oxycarbure de silicium, le carbure de titane, l'oxyde d'aluminium, le nitrure d'aluminium, l'oxyde de zirconium et les multicouches de ceux-ci ; le carbone adamantin ; le diamant ; le verre ; les polymères basse perméabilité et/ou denses, y compris les fluorocarbones, le polyuréthane, le PEEK, le silicone, le PDMS, le parylène, le polyimide ou les multicouches de ceux-ci.

19. Procédé selon l'une quelconque des revendications 15 à 18, dans lequel la couche amovible (112) comprend ou est constituée d'un matériau choisi parmi un matériau polymère, de préférence une résine, plus préférablement une résine photosensible et un matériau polymère soluble.

20. Procédé selon l'une quelconque des revendications 15 à 19, dans lequel le revêtement supérieur (107) est biocompatible,
et/ou
dans lequel le revêtement supérieur (107) est résistant à la corrosion,
et/ou
dans lequel le revêtement supérieur (107) est transparent,
et/ou
dans lequel le revêtement supérieur (107) comprend ou est constitué d'un matériau choisi parmi les céramiques ; le verre, y compris le SiC PECVD, le SiOC, le SiO₂ ; le diamant ou le carbone adamantin ; les oxydes d'aluminium ; les oxydes de titane ; ou les multicouches de ceux-ci.

21. Procédé selon l'une quelconque des revendications 13 à 20, dans lequel le procédé fournit un dispositif enrobé selon l'une quelconque des revendications 1 à 11.
